(19) 

Europäisches
Patentamt
European
Patent Office
Office européen
des brevets

(11) **EP 4 517 758 A1**

(12) **EUROPEAN PATENT APPLICATION**
published in accordance with Art. 153(4) EPC

(43) Date of publication:
**05.03.2025 Bulletin 2025/10**

(21) Application number: **23796050.5**

(22) Date of filing: **07.04.2023**

(51) International Patent Classification (IPC):
**G16B 20/00** (2019.01)   **G16B 25/10** (2019.01)
**G16H 50/30** (2018.01)

(52) Cooperative Patent Classification (CPC):
**G16B 20/00; G16B 25/10; G16H 50/30**

(86) International application number:
**PCT/JP2023/014370**

(87) International publication number:
**WO 2023/210304 (02.11.2023 Gazette 2023/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.04.2022  US 202263335943 P
22.08.2022  JP 2022131876**

(71) Applicant: **Japan Science and Technology
Agency**
**Kawaguchi-shi, Saitama 332-0012 (JP)**

(72) Inventors:
• **CHEN, Luonan**
  **Tokyo 113-0033 (JP)**
• **LI, Peiluan**
  **Luoyang 471023 (CN)**
• **AIHARA, Kazuyuki**
  **Tokyo 113-0033 (JP)**

(74) Representative: **Noble, Nicholas et al**
**Kilburn & Strode LLP**
**Lacon London**
**84 Theobalds Road**
**London WC1X 8NL (GB)**

(54) **DEVICE FOR DETECTING PREDICTOR OF ABRUPT CHANGE IN SYSTEM STATE, METHOD FOR DETECTING PREDICTOR OF ABRUPT CHANGE IN SYSTEM STATE, PROGRAM FOR DETECTING PREDICTOR OF ABRUPT CHANGE IN SYSTEM STATE, CONGESTION PREDICTOR DETECTION DEVICE, CONGESTION PREDICTOR DETECTION METHOD, CONGESTION PREDICTOR DETECTION PROGRAM, PRE-DISEASE STATE DETECTION DEVICE, PRE-DISEASE STATE DETECTION METHOD, PRE-DISEASE STATE DETECTION PROGRAM, AND RECORDING MEDIUM**

(57)   An apparatus for detecting signs of a sudden change in system state 2, comprising: a classifier 11 which classifies a plurality of nodes into a plurality of clusters based on the correlation of time-series changes of measurement data concerning a plurality of nodes constituting the target system; and a switch 12 which selects a cluster that satisfies predefined selection conditions based on the time-series changes of measurement data of the nodes in each classified cluster and the correlation of time-series changes of measurement data among all the nodes and switches between detecting a node in the selected cluster as the dynamical network marker that can be used as predictors of sudden state changes and detecting a specific node that has been stored in advance as the dynamical network marker.

FIG. 14

**Description**

TECHNICAL FIELD

This disclosure relates to an apparatus for detecting signs of a sudden change in system state, a method for detecting signs of a sudden change in system state, a program for detecting signs of a sudden change in system state, an apparatus for detecting signs of traffic congestion, a method for detecting signs of traffic congestion, a program for detecting signs of traffic congestion, an apparatus for detecting pre-disease state, a method for detecting pre-disease state, a program for detecting pre-disease state and a recording medium.

BACKGROUND ART

In many complex systems, such as biological systems, transportation systems, economic systems, etc., it is known that a sudden transition from one stable state, also referred to as a "steady state", to the next stable state, hereinafter referred to as a "predictive sign of sudden state change", occurs when a state reaches a certain critical threshold, hereinafter also referred to as a "tipping point", see, for example, Non-Patent Literature 1-5. If it is possible to detect the tipping point at which a sudden transition from one stable state to the next stable state, hereinafter referred to as a "predictive sign of sudden state change", it is expected to be possible to make predictions with regard to changes in the state of the system, for example, early detection and early treatment of diseases and prediction of traffic congestion, etc.

An example of known studies on the dynamic mechanism of complex diseases includes one that derives the relationship between state transitions at tipping points and rapid deterioration of diseases by modeling the process of disease deterioration, e.g., asthma attack, cancer development, etc., as a time-dependent nonlinear dynamical system and by analyzing it. See, for example, Non-Patent Literature 6.

Against this background, a dynamical network marker (Dynamical Network Marker, hereinafter also referred to as "DNM"), which is an indicator of the sudden onset of symptoms of a condition of a living body, i.e., unwellness, has been developed. In particular, in the case of a living body, a technology for detecting (Dynamical Network Biomarker, which is also called "DNB") has been disclosed, see, for example, Patent Literature 1. Such DNMs are based on measurement data of multiple factor items obtained from measurements on living organisms. Furthermore, a technology for detecting DNM based on this local network entropy is disclosed, see, for example, Patent Literature 2. Furthermore, there is disclosed a technology that stores the data of biological substances collected and analyzed from multiple healthy humans, adds the analysis data of biological substances collected only once from a subject to it, and determines whether DNM can be detected or not, see, for example, Patent Literature 3.

RELATED-ART LITERATURE

PATENT LITERATURE

Patent Literature 1: JP 2014-64515
Patent Literature 2: JP 2014-83194
Patent Literature 3: WO 2018/207925

NON PATENT LITERATURE

Non Patent Literature 1: Jose G. Venegas, Tilo Winkler, Guido Musch, Marcos F. Vidal Melo, Dominick Layfield, Nora Tgavalekos, Alan J. Fischman, Ronald J. Callahan, Giacomo Bellani, and R. Scott Harris,"Self-organized patchiness in asthma as a prelude to catastrophic shifts" Nature 434, Nature Publishing Group, pp. 777-782 (2005) Non Patent Literature 2: Patrick E. McSharry, Leonard A. Smith, and Lionel Tarassenko,"Prediction of epileptic seizures: are nonlinear methods relevant?" Nature Medicine 9, Nature Publishing Group, pp. 241-242 (2003)
Non Patent Literature 3: Roberto Pastor-Barriuso, Eliseo Guallar, and Josef Coresh,"Transition models for change-point estimation in logistic regression" Statistics in Medicine 22(7), Wiley-Blackwell, pp. 1141-1162 (2003)
Non Patent Literature 4: Paek SH et al. "Hearing preservation after gamma knife stereotactic radiosurgery of vestibular schwannoma" Cancer 104, Wiley-Blackwell, pp. 580-590 (2005)
Non Patent Literature 5: Liu, J.K., Rovit, R.L., and Couldwell, W.T., "Pituitary Apoplexy" Seminars in Neurosurgery 12, Thieme, pp. 315-320 (2001)

Non Patent Literature 6: G.Tanaka, K.Tsumoto, S.Tsuji, K.Aihara, "Bifurcation analysis on a hybrid systems model of intermittent hormonal therapy for prostate cancer" Physica D: Nonlinear Phenomena Volume 237, Issue 20, 15 October 2008, pp. 2616-2627

Non Patent Literature 7: Md Abdus Samad Kamal1, Makito Oku, Tomohisa Hayakawa, Jun-ichi Imura, Kazuyuki Aihara, "Early Detection of a Traffic Flow Breakdown in the Freeway Basedon Dynamical Network Markers" International Journal of Intelligent Transportation Systems Research (2020) 18:422.435

## SUMMARY OF INVENTION

### TECHNICAL PROBLEM

The technique disclosed in Patent Literature 1 detects DNMs using the Pearson correlation coefficient index. This technique has the issue of weak tolerance to noise. On the other hand, the technology disclosed in Patent Literature 2 detects DNMs using an index of network entropy. This technique has improved noise immunity compared to the one in the Patent Literature, but it is still not sufficient. The technology disclosed in Patent Literature 3 detects DNMs from a network of measurement data with one sample added using an index of differential correlation coefficients. However, both methods require a large amount of measurement and calculation of indices because all factor items are measured each time.

The purpose of this invention is to make it possible to avoid the enormous amount of calculation required to detect signs of a sudden change in the state of the target system.

### SOLUTION TO PROBLEM

In order to solve the above problem, an apparatus for detecting signs of a sudden change in system state according to an embodiment of the present invention comprises:

a classifier which classifies a plurality of nodes into a plurality of clusters based on the correlation of time-series changes of measurement data concerning a plurality of nodes constituting the target system; and
a switch which selects a cluster that satisfies predefined selection conditions based on the time-series changes of measurement data of the nodes in each classified cluster and the correlation of time-series changes of measurement data among all the nodes and switches between detecting a node in the selected cluster as the dynamical network marker that can be used as predictors of sudden state changes or detecting a specific node that has been stored in advance as the dynamical network marker.

Another embodiment of the present invention relates to a method for detecting signs of a sudden change in system state. The method comprises:

a classifying step to classify a plurality of nodes into a plurality of clusters based on the correlation of time-series changes of measurement data concerning a plurality of nodes constituting the target system; and
a switching step to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

Yet another embodiment of the present invention relates to a program for detecting signs of a sudden change in system state. The program causes a computer to perform:

a classifying step to classify a plurality of nodes into a plurality of clusters based on the correlation of time-series changes of measurement data concerning a plurality of nodes constituting the target system; and
a switching step to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

Yet another embodiment of the present invention relates to a recording medium which records a program for detecting signs of a sudden change in system state which causes a computer to perform:

a classifying step to classify a plurality of nodes into a plurality of clusters based on the correlation of time-series changes of measurement data concerning a plurality of nodes constituting the target system; and

a switching step to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

Yet another embodiment of the present invention relates to an apparatus for detecting signs of traffic congestion on the road. The apparatus comprises:

a dynamical network marker detector which detects a dynamical network marker based on the dispersion of traffic volume at each node and the correlation of traffic volume among the nodes, where multiple points on the road are nodes; and

a switch which switches between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

Yet another embodiment of the present invention relates to a method for detecting signs of traffic congestion on the road. The method comprises:

a dynamical network marker detection step to detect a dynamical network marker based on the dispersion of traffic volume at each node and the correlation of traffic volume among the nodes, where multiple points on the road are nodes; and

a switching step to switch between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

Yet another embodiment of the present invention relates to a program for detecting signs of traffic congestion on the road. The program causes a computer to perform:

a dynamical network marker detection step to detect a dynamical network marker based on the dispersion of traffic volume at each node and the correlation of traffic volume among the nodes, where multiple points on the road are nodes; and

a switching step to switch between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

Yet another embodiment of the present invention relates to a recording medium which records a program for detecting signs of traffic congestion on the road which causes a computer to perform:

a dynamical network marker detection step to detect a dynamical network marker based on the dispersion of traffic volume at each node and the correlation of traffic volume among the nodes, where multiple points on the road are nodes; and

a switching step to switch between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

Yet another embodiment of the present invention relates to an apparatus for detecting pre-disease state which is the turning point from the normal state to the disease state. The apparatus comprises:

a data generator which generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculator which calculates a reference sample network from the reference gene expression data and a perturbation sample network from the gene expression data for testing;

a sample specific network calculator which calculates a sample specific network which represents the relationship between the reference sample network and the perturbation sample network;

a sample specific differential network calculator which calculates a sample specific differential network from the sample specific network at the first time and the sample specific network at the second time after the first time;

a local network extractor which extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculator which calculates a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculator which calculates a differential network flow entropy from the local network flow entropy and the conditional network flow entropy;

a temporal differential network flow entropy calculator which calculates a temporal differential network flow entropy from the differential network flow entropy; and

a pre-disease state detector,

wherein the pre-disease state detector detects a pre-disease state when the difference between the temporal differential network flow entropy at the first time and the temporal differential network flow entropy at the second time is more than a predetermined value.

The gene expression probability may be calculated assuming that the expression of each adjacent gene follows a normal distribution.

The reference gene expression data may be an m-by-n matrix such that the (i, j) component represents the expression level of the i-th (i=1, ..., m) gene at a time point in the past or of a person in the j-th (j=1, ..., n) subject and the gene expression data for testing may be an m-by-n+1 matrix such that (i, j) component represents the expression level of the i-th (i=1, ..., m) gene at a time point in the past or of a person in the j-th (j=1, ..., n) subject and (i, n+1) component represents the expression level of the i-th (i=1, ..., m) of the subject,

where the number of the time points or the number of the persons is n and the number of gene types is m.

The reference sample network may be a Weighted Reference Network generated from the reference gene expression data and the perturbation sample network may be a Weighted Perturbation Network generated from the gene expression data for testing.

The sample specific network may be calculated by comparing the reference sample network with the perturbation sample network and leaving only the different edge.

The sample specific differential network may be calculated by comparing the sample specific network at the first time and the sample specific network at the second time and leaving only the different edge.

The gene expression probability $p(x_{ikj})$ may be: [equation 8]

$$p\left(x_{i_k j}\right) = \frac{\dfrac{1}{\sqrt{2\pi\sigma^2}} \int_{-\infty}^{x_{i_k j}} e^{\dfrac{-\left(t-\mu_{i_k}\right)^2}{2\left(\sigma_{i_k}\right)^2}} dt}{\sum_{j=1}^{n+1} \dfrac{1}{\sqrt{2\pi\sigma^2}} \int_{-\infty}^{x_{i_k j}} e^{\dfrac{-\left(t-\mu_{i_k}\right)^2}{2\left(\sigma_{i_k}\right)^2}} dt} \qquad (14)$$

where $x_{ikj}$ represents the (ik, j) component of the expression data of the i-th gene $g_i{}^k$ in the k-th local network and where $\mu_{ik}$ and $\sigma_{ik}$ are the mean deviation and the standard deviation of gene $g_i{}^k$, respectively.

The local network flow entropy $NFE_T(x_{ik})$ may be: [equation 5]

$$NFE_T\left(x_{i_k}\right) = -\sum_{j=1}^{n+1} x_{i_k j}\, p\left(x_{i_k j}\right) \log\left(x_{i_k j} p\left(x_{i_k j}\right)\right) \qquad (1\,1)$$

and the conditional network flow entropy $NFE_T\,(x_{ik}/x_k)$ may be: [equation 9]

$$NFE_T\left(x_{i_k}/x_k\right) = -\sum_{j=1}^{n+1} x_{i_k j}\, p\left(x_{kj}, x_{i_k j}\right) \log\left(x_{i_k j} p\left(x_{i_k j}/x_{kj}\right)\right) \qquad (1\,5)$$

where $x_{ik}$ is the expression data of the i-th gene $g_i{}^k$ in the k-th local network and
where $x_k$ is the expression data across genes in the k-th local network.

The differential network flow entropy $DNFE_T{}^k$ may be:
[equation 12]

$$DNFE_T{}^k = \frac{\sum_{i=1}^{M_k}\left(NFE_T\left(x_{i_k}\right) - NFE_T\left(x_{i_k}/x_k\right)\right)}{M_k} \qquad (1\,8)$$

where $M_k$ is the number of the adjacent genes.
The temporal differential network flow entropy TNFET may be:
[equation 13]

$$TNFE_T = \frac{\sum_{k=1}^{l} DNFE_T{}^k}{l} \qquad (1\,9)$$

where l is the number of the local networks.
The pre-disease state detector may detect a pre-disease state when the temporal differential network flow entropy at the second time is twice or more than the temporal differential network flow entropy at the first time.
Yet another embodiment of the present invention relates to a method for detecting pre-disease state which is the turning point from the normal state to the disease state. The method comprises:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculation step to calculate a reference sample network from the reference gene expression data and a perturbation sample network from the gene expression data for testing;
a sample specific network calculation step to calculate a sample specific network which represents the relationship between the reference sample network and the perturbation sample network;
a sample specific differential network calculation step to calculate a sample specific differential network from the sample specific network at the first time and the sample specific network at the second time after the first time;
a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;
a gene expression probability calculation step to calculate a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and a pre-disease state detection step,

wherein the pre-disease state detection step detects a pre-disease state when the difference between the temporal differential network flow entropy at the first time and the temporal differential network flow entropy at the second time is more than a predetermined value.

Yet another embodiment of the present invention relates to a program for detecting pre-disease state which is the turning point from the normal state to the disease state. The program causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detection step detects a pre-disease state when the difference between the temporal differential network flow entropy at the first time and the temporal differential network flow entropy at the second time is more than a predetermined value.

Yet another embodiment of the present invention relates to a recording medium which records a program for detecting pre-disease state which is the turning point from the normal state to the disease state which causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;
a differential network flow entropy calculation step to calculate a differential network flow entropy from the local network flow entropy and the conditional network flow entropy;
a temporal differential network flow entropy calculation step to calculate a temporal differential network flow entropy from the differential network flow entropy; and
a pre-disease state detection step,
wherein the pre-disease state detection step detects a pre-disease state when the difference between the temporal differential network flow entropy at the first time and the temporal differential network flow entropy at the second time is more than a predetermined value.

Yet another embodiment of the present invention relates to an apparatus for detecting pre-disease state which is the turning point from the normal state to the disease state. The apparatus comprises:

a data generator which generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculator which calculates a reference sample network from the reference gene expression data and a perturbation sample network from the gene expression data for testing;
a sample specific network calculator which calculates a sample specific network which represents the relationship between the reference sample network and the perturbation sample network;
a sample specific differential network calculator which calculates a sample specific differential network from the sample specific network at the first time and the sample specific network at the second time after the first time;
a local network extractor which extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;
a gene expression probability calculator which calculates a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;
a differential network flow entropy calculator which calculates a differential network flow entropy from the local network flow entropy and the conditional network flow entropy;
a temporal differential network flow entropy calculator which calculates a temporal differential network flow entropy from the differential network flow entropy; and
a pre-disease state detector,
wherein the pre-disease state detector comprises a switching means which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

Yet another embodiment of the present invention relates to a method for detecting pre-disease state which is the turning point from the normal state to the disease state. The method comprises:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculation step to calculate a reference sample network from the reference gene expression data and a perturbation sample network from the gene expression data for testing;
a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detector comprises a switching means which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

Yet another embodiment of the present invention relates to a program for detecting pre-disease state which is the turning point from the normal state to the disease state. The program causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detector comprises a switching means which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

Yet another embodiment of the present invention relates to a recording medium which records a program for detecting pre-disease state which is the turning point from the normal state to the disease state which causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detector comprises a switching means which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

Any combination of the above components, and any transformation of the expression of the present invention among devices, methods, systems, recording media, computer programs, etc., is also valid as an aspect of the present invention.

ADVANTAGEOUS EFFECTS OF INVENTION

According to the present invention, it is possible to avoid the enormous amount of calculations required to detect signs of sudden changes in the state of the target system.

BRIEF DESCRIPTION OF DRAWINGS

[FIG. 1] Fig. 1 shows a schematic diagram of the process of disease progression;
[FIG. 2] Fig. 2 shows a schematic diagram of the confluence road in the traffic simulator used for the study;
[FIG. 3] Fig. 3 shows a simulation results of the average traffic speed at the confluence of arterial roads in Case I;
[FIG. 4] Fig. 4 shows a simulation results of the traffic density at the confluence of arterial roads in Case I;
[FIG. 5] Fig. 5 shows a simulation results of the average traffic speed at the confluence of arterial roads in Case II;
[FIG. 6] Fig. 6 shows a simulation results of the traffic density at the confluence of arterial roads in Case II;
[FIG. 7] Fig. 7 is a Schematic diagram of the nine cells on an arterial road;
[FIG. 8] Fig. 8 shows the average of the vehicle density for all nodes in Case I;
[FIG. 9] Fig. 9 shows the standard deviation of the normalized measurements of the vehicle density for all nodes in Case I;
[FIG. 10] Fig. 10 shows the average of the vehicle density for all nodes in Case II;

[FIG. 11] Fig. 11 shows the standard deviation of the normalized measurements of the vehicle density for all nodes in Case II;

[FIG. 12] Fig. 12 shows the mean of the absolute values of the Pearson correlation coefficients (Cdd) within the set of dominant nodes in Case I;

[FIG. 13] Fig. 13 shows the mean of the absolute values of the Pearson correlation coefficients (Cdd) within the set of dominant nodes in Case II;

[FIG. 14] Fig. 14 shows a functional block diagram of the apparatus for detecting signs of a sudden change in system state according to the first embodiment;

[FIG. 15] Fig. 15 shows a flowchart of the method for detecting signs of a sudden change in system state according to the second embodiment;

[FIG. 16] Fig. 16 shows a functional block diagram of the apparatus for detecting signs of traffic congestion according to the fifth embodiment;

[FIG. 17] Fig. 17 shows a flowchart of the method for detecting signs of traffic congestion according to the sixth embodiment;

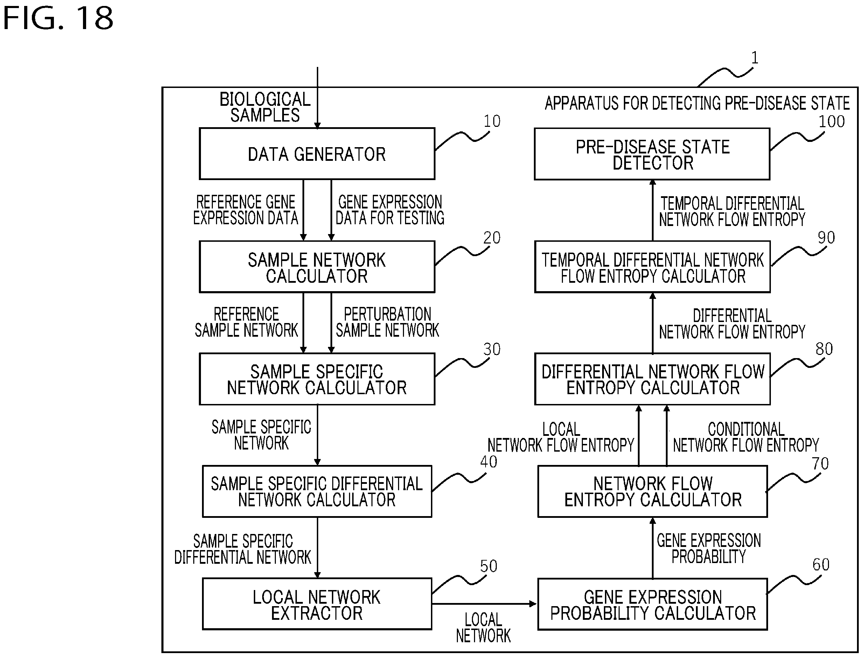

[FIG. 18] Fig. 18 shows a functional block diagram of the apparatus for detecting pre-disease state according to the ninth embodiment;

[FIG. 19] Fig. 19 shows a schematic diagram showing the operation of the data generator;

[FIG. 20] Fig. 20 shows a schematic diagram showing the operation of the sample network calculator;

[FIG. 21] Fig. 21 shows a schematic diagram showing the operation of the sample specific network calculator;

[FIG. 22] Fig. 22 shows a schematic diagram showing the operation of the sample specific differential network calculator;

[FIG. 23] Fig. 23 shows a schematic diagram showing the operation of the local network extractor;

[FIG. 24] Fig. 24 shows a flowchart of the method for detecting pre-disease state according to the tenth embodiment;

[FIG. 25] Fig. 25 shows a functional block diagram of the apparatus for detecting pre-disease state according to the thirteenth embodiment;

[FIG. 26] Fig. 26 shows a flowchart of the method for detecting pre-disease state according to the fourteenth embodiment;

## DESCRIPTION OF EMBODIMENTS

The disclosure will be explained below with reference to drawings based on suitable embodiments. The embodiments are examples rather than limitations of the disclosure. All features or combinations of features described in the embodiments are not necessarily essential to the disclosure. Identical or equivalent components, parts, and processes shown in each drawing shall be given the same symbol and duplicate explanations will be omitted where appropriate. The scale and shape of each part shown in each drawing are set for convenience in order to facilitate explanation and should not be construed as limiting unless otherwise noted. When terms such as "first", "second", etc. are used in this specification or in the claims, unless otherwise mentioned, such terms do not indicate any order or degree of importance, but only to distinguish one configuration from another. In addition, in each drawing, some parts of the components that are not important in explaining the embodiments are omitted.

Basic findings are explained before describing the specific embodiments.

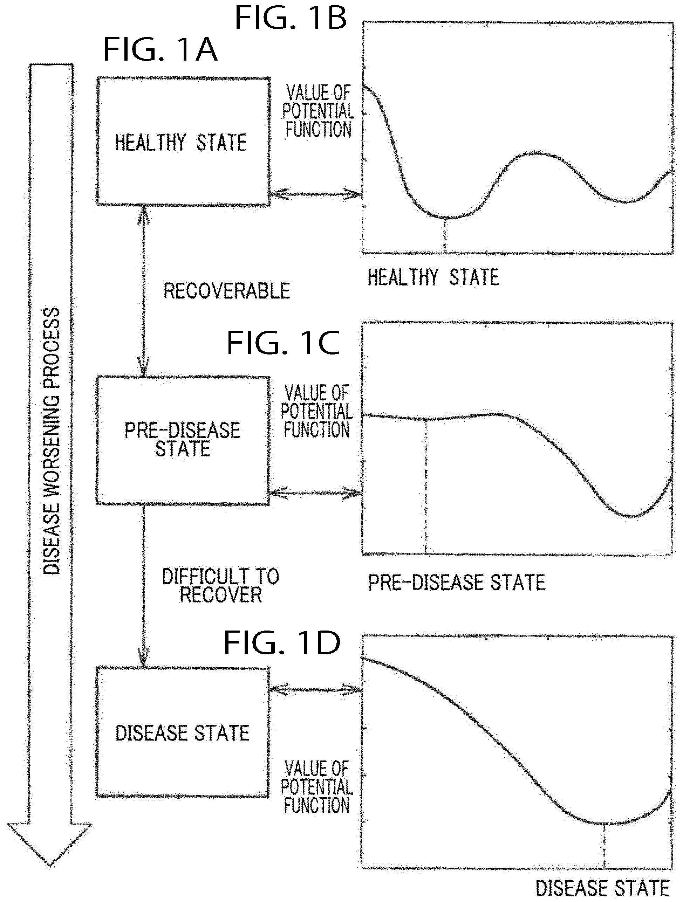

Figure 1 schematically shows the process of the state of a system. For example, Figure 1 a schematically shows the process of disease progression. Figures 1 b, c and d show the stability of the nonlinear dynamics system in the course of the progression process by potential functions. In Figures 1 b, c and d, the values of the state variables of the system are taken on the horizontal axis and the values of the potential functions on the vertical axis. As shown in Figure 1 a, the progression process of disease aggravation can be represented using three state transitions: healthy state, pre-disease state and disease state. In the normal state, the system is stable. In this case, the value of the potential function takes a minimum value, as shown in Figure 1 b. In the pre-disease state, the potential function of the system takes a high value, as shown in Figure 1 c. At this time, the system is susceptible to disturbances. Namely, the state of the system at this time is at a tipping point, in other words, at the limit of the healthy state, where only a small disturbance can cause the system to transition to the diseased state. However, such a pre-disease state can often be restored to a normal state by taking appropriate measures. In the diseased state, on the other hand, the system stabilizes again. That is, the value of the potential function again takes a minimum value, as shown in d in Figure 1. Therefore, once the system is in a diseased state, it is difficult to recover to a normal state. Usually, the term "pre-disease" means "a state in which a person has not yet developed a disease, but is moving away from a normal state." However, from the viewpoint of "a turning point in the transition from a normal state to a diseased state," the "pre-disease state" has extremely remarkable characteristics and is the key to early detection and early treatment of diseases.

Therefore, appropriate measures can be taken to return the patient to a healthy state if the pre-disease state can be detected before falling into the diseased state. Thus, for the early detection and early treatment of disease, it is extremely important to detect the pre-disease state which is the turning point from the normal state to the disease state.

Underlying Theory

Using genomic high-throughput technology, which can obtain a large number, e.g., thousands, of data, i.e., highdimensional data, from a single sample, the inventors constructed a mathematical model of the time evolution of complex diseases based on branching theory and studied the progression of disease deterioration at the molecular network level. As a result, they found the existence of DNMs, dynamical network markers, that can detect the immediate prebranching, **i.e.,** sudden deterioration, state before the onset of state transitions in the pre-disease state. If this DNM can be used as a warning signal for the pre-disease state, a disease model will become unnecessary, and early diagnosis of complex diseases can be realized with only a few samples.

The system for the disease progression process, hereinafter referred to as "system (1)", shall be expressed by the following equation (1).

$$Z(k+1) = f(Z(k); P) \quad (1).$$

$Z(k) = (z1(k), ..., zn(k))$ is a variable representing the dynamic state of system (1) observed at time k (k = 0, 1, ...), specifically, information such as protein expression levels, gene expression levels, metabolite expression levels, e.g., concentration and number of molecules of proteins, genes, etc. P is a slowly changing parameter that drives the state transition of the system (1) and can be information such as genetic factors such as SBP and CNV, and non-genetic factors such as methylation and acetylation. $f = (f1,..., fn) ..., fn)$ is a nonlinear function of Z(k).

The normal state and the disease state can be represented by the immobile point attractor of the state equation Z(k+1) = f(Z(k); P), respectively. The function f is a nonlinear function with thousands of variables because the process of complex disease progression has very complex dynamic characteristics. In addition, the elements of the parameter P that drive the system (1) are difficult to specify. Therefore, it is very difficult to construct and analyze system models of the normal state and the disease state.

The system (1) has immovable points which have the following characteristics (A1) to (A3).

(A1) $Z^* = f(Z^*; P)$, where $Z^*$ is the immovable point of system (1).

(A2) If Pc is the threshold at which the system bifurcates, and P = Pc, then the absolute value of one real eigenvalue or a pair of complex conjugate eigenvalues of the Jacobi matrix $\partial f(Z; Pc)/\partial Z|Z = Z^*$ is 1.

(A3) In general, when $P \neq Pc$, the absolute value of the eigenvalue of system (1) is not 1.

Based on the above characteristics, the inventors theoretically clarified that the following peculiar characteristics appear when system (1) is near the state transition point. That is a dominant group, i.e., subnetwork, consisting of some nodes appears in the network (1) which is composed of each variable z1, ..., zn in the system (1) as a node when the state of the system (1) is near the transition point. The dominant group that appears near the state transition point ideally has the specific characteristics shown in (B1)-(B3) below.

(B1)

$$PCC(z_i, z_j) \rightarrow \pm 1;$$

$$SD(z_i) \rightarrow \infty;$$

$$SD(z_j) \rightarrow \infty;$$

if $z_i$ and $z_j$ are nodes belonging to the dominant group.

(B2)

$$PCC(z_i, z_j) \rightarrow 0;$$

$$SD(z_i) \rightarrow \infty;$$

$$SD(z_j) \rightarrow \text{bounded value};$$

if $z_i$ is a node belonging to the dominant group but $z_j$ is not a node belonging to the dominant group.

(B3)

$$PCC(z_i, z_j) \rightarrow \alpha, \alpha \in (-1, 1);$$

$$SD(z_i) \rightarrow \text{bounded value;}$$

$$SD(z_j) \rightarrow \text{bounded value;}$$

if $z_i$ and $z_j$ are not nodes belonging to the dominant group.

$PCC(z_i, z_j)$ is the Pearson correlation coefficient between $z_i$ and $z_j$. $SD(z_i)$ and $SD(z_j)$ are the standard deviations of $z_i$ and $z_j$, respectively.

In other words, in network (1), the appearance of a dominant group with the above specific characteristics (B1) to (B3) can be taken as a sign that system (1) is in a critical transition state, i.e., pre-disease state. Therefore, by detecting the dominant group, the signs of a state transition of the system (1) can be detected. In other words, the dominant group can be considered as a warning signal that indicates a state transition, or in other words, a pre-disease state just before the disease worsens. Therefore, no matter how complex the system (1) is or how unknown the driving parameter elements are, by detecting only the dominant groups that serve as warning signals, the pre-disease state can be identified without directly dealing with the mathematical model of the system (1). It is possible to realize advance countermeasures and early treatment against diseases by identifying the pre-disease state. As disclosed in Patent Literature 1, the inventors have named the relevant dominant group, which is a warning signal for such pre-disease state, as "DNM", dynamical network marker, and in particular "DNB", dynamical network biomarker, when targeting biological systems, and proposed

$$I = SDd \times PCCd/OPCCd$$

as an overall index for detecting them, where

SDd: Mean value of the standard deviation of the nodes in the DNM,
PCCd: Mean value of the absolute value of the Pearson correlation coefficient between nodes in the DNM and
OPCCd: the average of the absolute values of the Pearson correlation coefficients between a node and other nodes in the DNM.

In other words, this DNM is a network that shows a logical dynamic coupling relationship that produces effective coupling only at specific times, and is used as a marker to indicate signs of sudden state changes.

Thus, DNM is a dominant group with specific characteristics (B1)-(B3), which appears in network (1) as a subnetwork comprising multiple nodes when the system (1) is in a pre-disease state. DNM is a group comprising factor items for some biomolecules that satisfy the above specific characteristics (B1) to (B3), i.e., subnetwork, if each node (z1, ..., zn) in network (1) is a factor item to be measured for biomolecules such as genes, proteins, metabolites, etc.

A method for detecting candidates for DNMs by directly utilizing the above specific characteristics (B1) to (B3) is disclosed in Patent Literature **1.** According to this method, it is possible to detect DNMs that warn of a transition to a disease state, from a biological sample. However, the detection accuracy deteriorates if the measurement data contains noise. In addition, the amount of calculation is enormous and the detection efficiency is not high because it is necessary to detect DNMs that satisfy the above conditions (B1) to (B3) from a large amount of measurement data.

Use of stored DNMs

In contrast, the inventors found that the nodes of the dominant group constituting the DNM for each disease are almost the same regardless of the organism, and that the huge amount of calculation required for DNM detection can be omitted if the above nodes are stored and (B1) to (B3) are calculated for them and if the DNM is judged to have been detected only when almost all nodes satisfy the conditions (B1) to (B3). In other words, the amount of calculation for PCC can be reduced to almost (the number of combinations when 2 are selected from n)/(the number of combinations when 2 are selected from N), where the total number of nodes is N and the number of nodes in the dominant group, **i.e.,** DNM, that have been stored is n.

In this case, (B1)-(B3) are as follows.

(B1)'

It is checked if the following conditions are satisfied for nodes $z_i$ and $z_j$ belonging to the 'dominant group.'

$$PCC(z_i, z_j) \to \pm 1;$$

$$SD(z_i) \to \infty;$$

$$SD(z_j) \to \infty;$$

The DNM detection is aborted as DNM is not detected if more than half of the nodes do not satisfy these conditions.

(B2)'

If (B1)' is satisfied, it is checked if the following conditions are satisfied for node $z_i$ belonging to the dominant group and $z_j$ not belonging to the dominant group. $PCC(z_i, z_j) \to 0$;

$$SD(z_i) \to \infty;$$

$$SD(z_j) \to \text{bounded value};$$

for the nodes $z_i$ and zj that do not belong to the dominant group. $SD(z_j)$ is not calculated and DNM detection is aborted as no DNM is detected if more than half of the nodes belonging to the dominant group do not satisfy these conditions.

(B3)'

If (B2)' is satisfied and if $z_i$ and $z_j$ are not nodes belonging to the dominant group,
it is checked if the following conditions are satisfied

$$PCC(z_i, z_j) \to \alpha, \alpha \in (-1, 1);$$

$$SD(z_i) \to \text{bounded value};$$

$$SD(z_j) \to \text{bounded value};$$

and it is checked if

$$I = SDd \times PCCd/OPCCd$$

exceeds the preset threshold value.
DNM is determined to have been detected if these conditions are satisfied.

However, there may be concerns about whether the stored DNMs can continue to be fixed and used due to foreseeable stochastic fluctuations of DNMs, gradual shift phenomena, and the like. For this reason, it is also effective to provide a switch that can switch the DNM detection target node so that the DNM detection can be performed for all nodes by returning to the original algorithm.

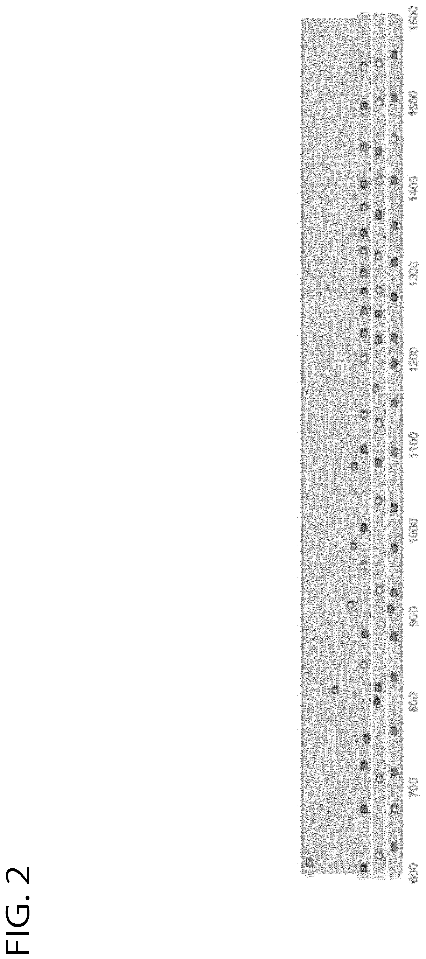

Application to Traffic Congestion Prediction Figure 2 shows a schematic of the confluence road in the traffic simulator used for the study. The arterial road has three lanes, with the leftmost lane connected by a 250 m ramp in the merging section. The minimum spacing between vehicles follows a lognormal distribution with mean 1.7 m, standard deviation 0.9 m, and minimum 1 m. The vehicle speeds follow a normal distribution with mean 100 km/h and standard deviation 7.5 km/h. The length of the vehicle is 4 m. The parameters related to acceleration and acceleration on deceleration are set to 1.5 m/s$^2$ and 2.0 m/s$^2$, respectively. The vehicle is assumed not to make two consecutive lane changes within 10 seconds. The time step for the simulation is 0.1 second.

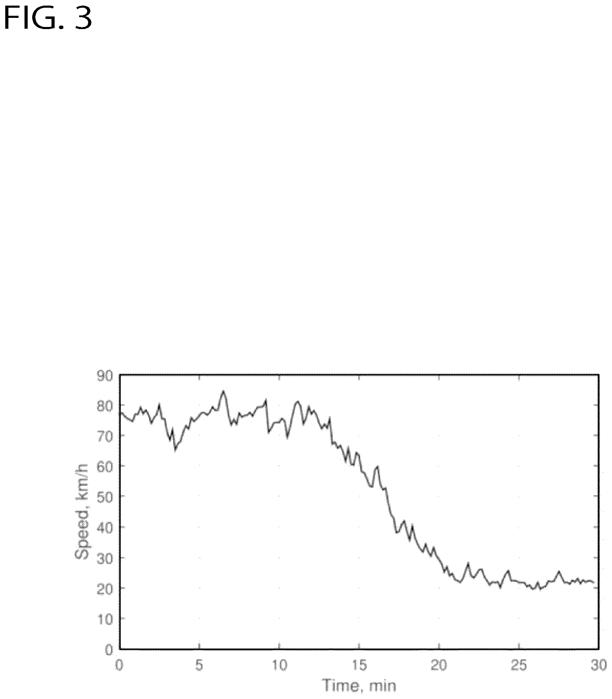

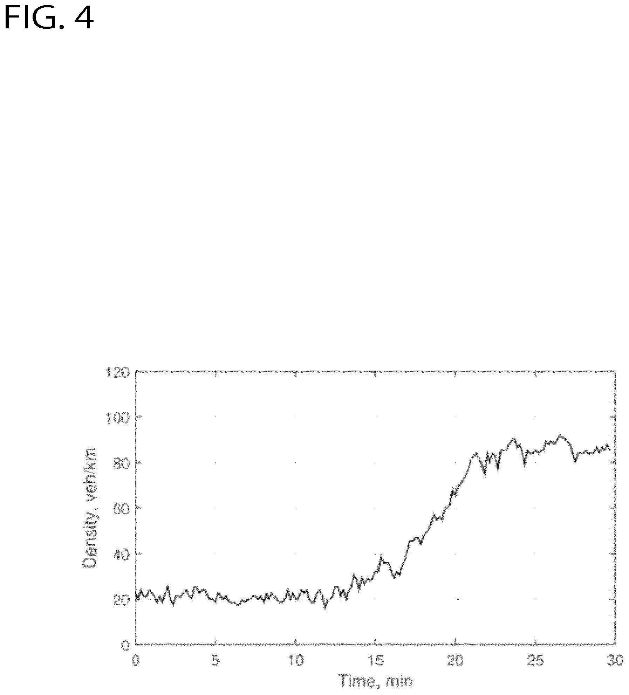

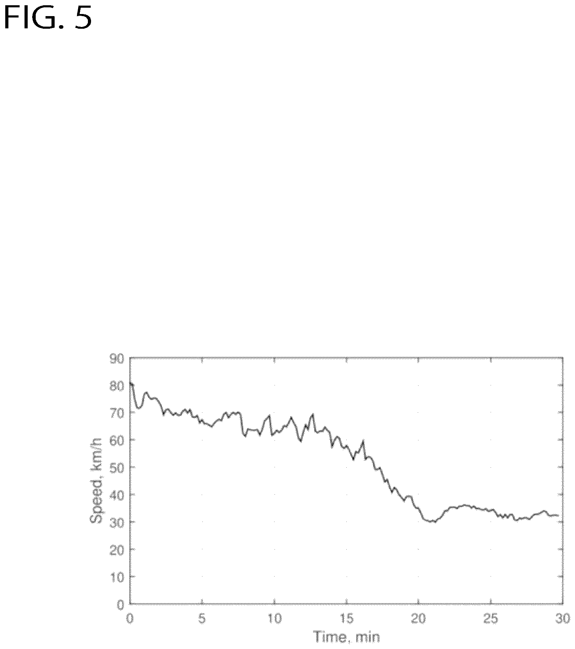

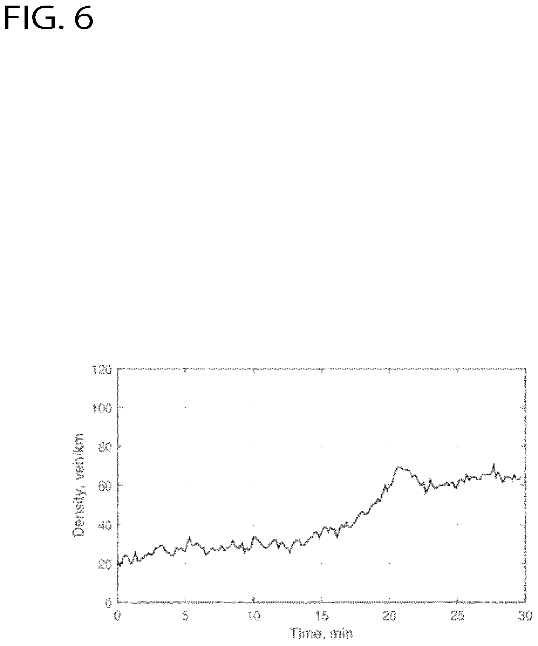

Two representative traffic flow scenarios are shown in Figures 3 through 6. Figures 3 and 4 show the speed and the traffic density in Case I, respectively. Figures 5 and 6 show the speed and the traffic density in Case II, respectively. In Case I, the incoming traffic on the arterial road was assumed to average 4,050 vehicles/hour and the traffic on the ramp was assumed to average 850 vehicles/hour. In Case II, the incoming traffic on the arterial road was assumed to average 4,800 vehicles/hour and the traffic on the ramp was assumed to average 320 vehicles/hour. The incoming traffic volume in Case II is slightly higher than in Case I, however the merging zone is relatively smoother because of the lower ramp traffic volume. Figures 3 and 4 show the simulated average traffic speed and traffic density in the merging zone of the arterial roadway in Case I, respectively. Figures 5 and 6 show the simulated average traffic speed and traffic density in the merging zone of the arterial roadway in Case II, respectively. In Case I, the traffic flow modulates after about 10 minutes, and finally the average traffic speed drops to about 22 km/h in the merging zone and the vehicle density exceeds 85 vehicles per km. Stop-and-go congestion is induced at the merging zone, leading to traffic congestion. On the other hand, in Case II, despite the slightly higher total traffic volume, a gradual decrease in speed occurs, however it does not lead to congestion.

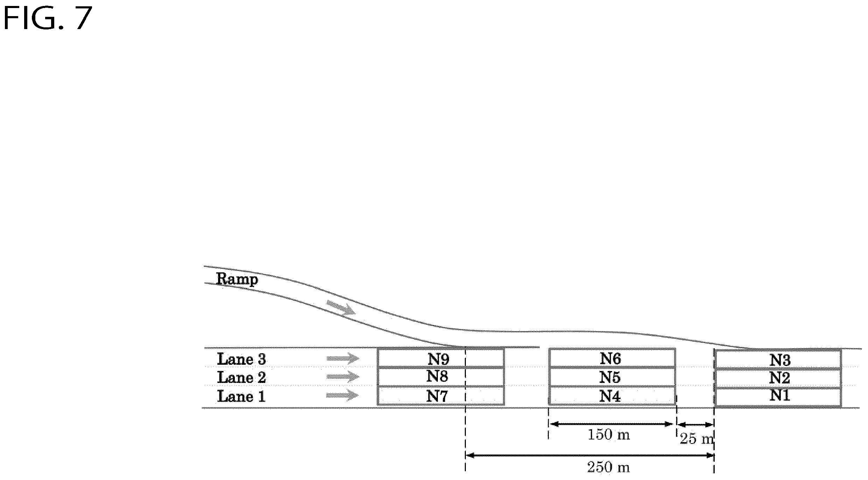

For the purpose of applying the present disclosure, a road is considered as a traffic network that is divided into cells connected to each other. Figure 7 shows a schematic of nine cells numbered N1 to N9 on an arterial road. These are nodes of the DNM. It should be noted that each lane cell is 150 m long. The first three nodes, N1-N3, are set at the edge of the confluence zone, and the next three nodes, N4-N6, are set parallel to the confluence zone, 25 m apart from the first set of nodes. The last three nodes, N7-N9, are set on the beginning side of the confluence zone at the same interval from the second set of nodes.

At each node, vehicle interval data is acquired every 0.5 seconds. The acquired data is normalized by mean and variance, and the normalized value z(k), where k is the node number, is used to calculate the standard deviation per node and the correlation between nodes, **i.e.,** Pearson correlation coefficient. Nodes N4 to N6 show a higher standard deviation than the other nodes and a very high correlation between N4 and N6 are presented, especially after 16 minutes, when the state is considered to be in transition. For this reason, these three nodes N4 to N6 are selected as the dominant set of nodes. The mean absolute values of the Pearson correlation coefficients between these nodes and the other nodes always remain low without significant changes at any stage.

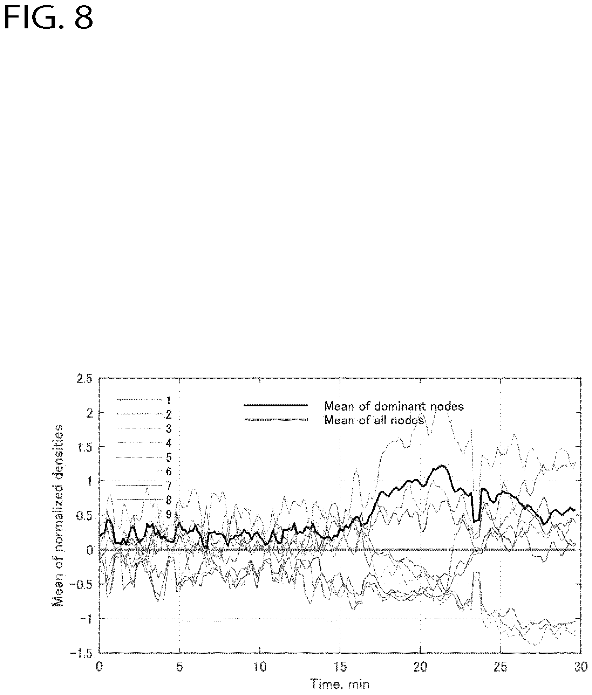

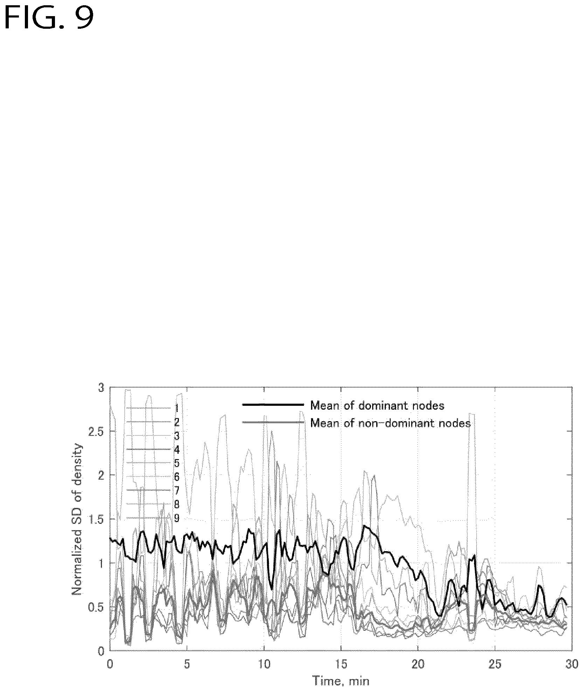

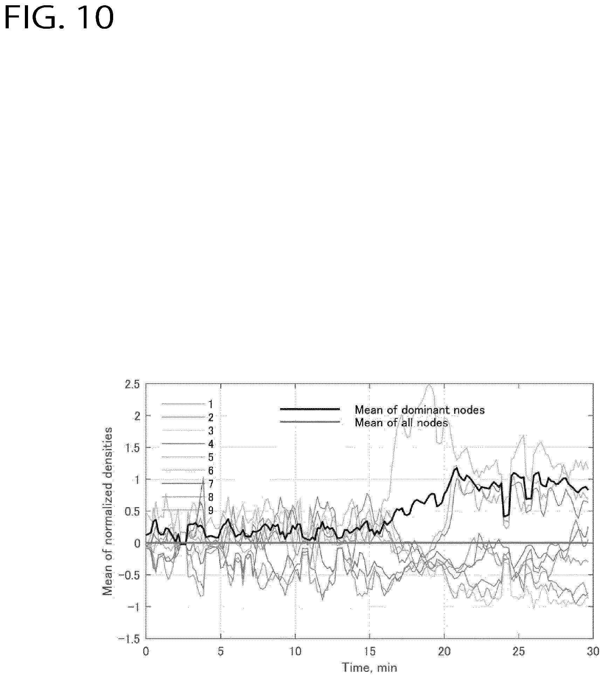

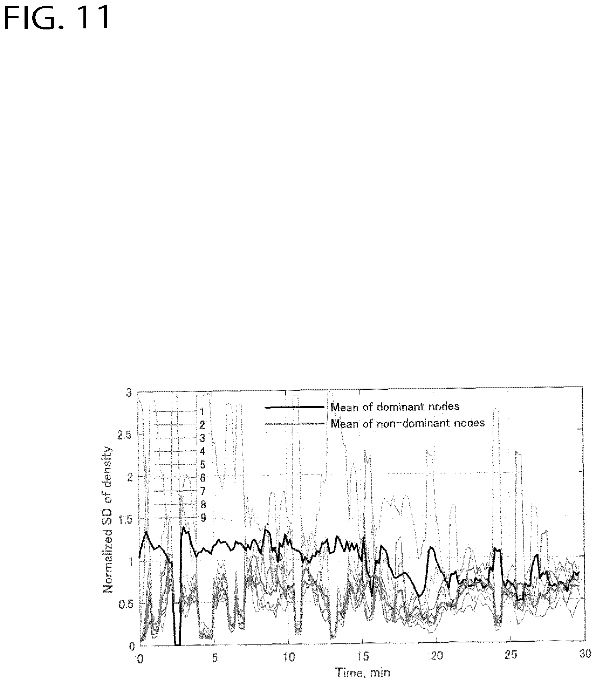

The effectiveness of the disclosure was evaluated in Case I and Case II. Figures 8 and 9 show the mean and the standard deviation of the normalized measured values of the vehicle density at all nodes in Case I, respectively. Figures 10 and 11 show the mean and the standard deviation of the normalized measured values of the vehicle density at all nodes in Case II, respectively. The means of the standard deviations of the measurements for the dominant and nondominant sets of nodes are also plotted in Figures 9 and 11. It can be seen that, generally, the dominant set of nodes always outperforms the non-dominant set of nodes. Especially in Case I, as shown in Figure 9, the mean standard deviation of the dominant set of nodes is clearly higher than the mean standard deviation of the non-dominant set of nodes from around 16 to 21 minutes. On the other hand, in Case II, as shown in Figure 11, no such explicit phenomenon can be observed.

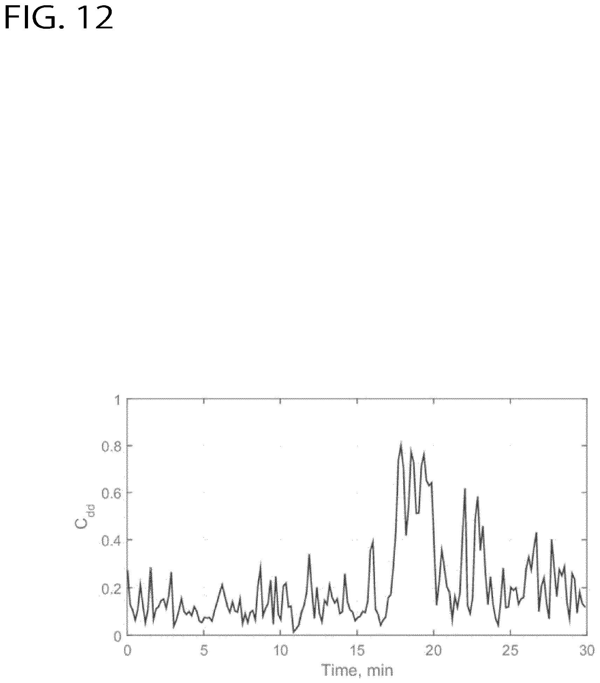

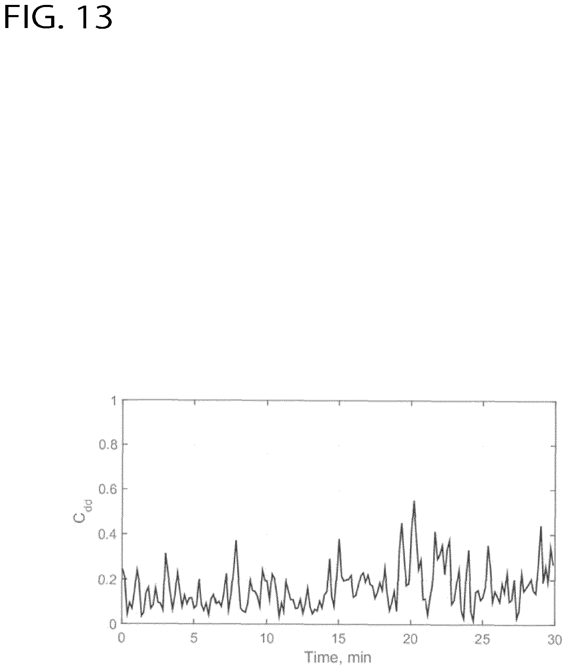

Figures 12 and 13 show the mean absolute value of the Pearson correlation coefficient (Cdd) within the dominant node set. In Case I, the value increases very rapidly from 16 to 21 minutes and then shows a decreasing trend. In Case II, however, no such phenomenon is observed. Thus, the index of DNM, which has the product of the mean of the standard deviation of the dominant set of nodes and the mean of the absolute value of the Pearson correlation coefficient within the dominant set of nodes in the numerator, increases rapidly from 16 to 21 minutes in Case I, whereas in Case II, no such phenomenon is observed. Figures 3 and 4 show that in Case I, the merging zone is completely congested after 22 minutes, and the congestion is even greater. Therefore, it can be concluded that in Case I, the DNM indicator can detect the signs of congestion. On the other hand, Figures 5 and 6 show that in Case II, although the vehicle density increases, it is not as high as in Case I, and the average vehicle speed is maintained at about 35 km/h, indicating that no obvious congestion occurs.

In the above, nodes N4 to N6 are selected as the dominant set of nodes. Therefore, it is necessary to calculate the Pearson correlation coefficients among all nodes (36 streets). Here, if we memorize in advance that N4 to N6 are likely to be the dominant set of nodes, there will be no need to calculate Pearson correlation coefficients for the set of dominant nodes and the set of nondominant nodes. This significantly reduces the amount of computation, because only three Pearson correlation coefficients within the set of dominant nodes need to be computed. However, the assumed conditions may change, such as changes in the location or structure of the merging ramps. For this reason, it is dangerous to always fix the dominant set of nodes to the memorized ones. Therefore, it is important to be able to switch the calculation process so that the Pearson correlation coefficient is calculated using all nodes instead of the memorized set of dominant nodes in order to check for changes in the assumptions from time to time.

Application to pre-disease detection

As a method that can detect DNM more accurately and efficiently, the inventors proposed a method for detecting pre-disease states using local network entropy based on transition states, see, for example, Patent Literature 2. The method is described in detail below. Local network entropy is the microscopic entropy calculated statistically focusing on a single node in a network that exhibits logical dynamic coupling relations that produce effective coupling only at specific times.

The use of local network entropy has improved immunity to noise compared to Patent Literature 1, however it is still not sufficient. Furthermore, the problem of requiring a large amount of measurement data has not been solved.

In order to solve this problem, the inventors have studied intensively and found a method to detect DNM more precisely and efficiently by using network flow entropy based on transition states to detect pre-disease states.

Network flow entropy based on transition states Dynamic behavior of the aforementioned system (1) can be approximated by the following equation (2) in the vicinity of a tipping point.

$$Z(t+1) = A(P)Z(t) + \varepsilon(t) \quad (2)$$

$\varepsilon(t)$ is the Gaussian noise and P is the parameter vector that controls the Jacobian A of the nonlinear function f in system (1).

Then, we can prove the following conclusions (C1) and (C2) based on branching theory and central manifold theory, where the change of Z is denoted $\Delta z_i(t) = z_i(t) - z_i(t-1)$ (i = 1, 2, ..., n).

(C1)

If P does not exist in the vicinity of a state transition point or a tipping point, for any node i, j (including the case i=j), $\Delta z_i(t+T)$ is statistically independent of $\Delta z_j(t)$ (i, j=1, 2, ..., n).

(C2)

If P exists in the vicinity of a state transition point or a tipping point,

- when both nodes i and j exist in the dominant group or DNM, the correlation between $\Delta z_i(t+T)$ and $\Delta z_j(t)$ is high,
- when neither node i nor j exists in the dominant group or DNM, $\Delta z_i(t+T)$ is statistically independent from $\Delta z_j(t)$.

Based on the above conclusions (C1) and (C2), the inventors focused on transition states and found that DNMs can be detected with higher accuracy and efficiency by using Network Flow Entropy, also called as "NFE". The concept of NFE based on transition states and the relationship between NFE and DNM are explained below.

Transition state

The transition state for an arbitrary variable $z_i$ at time t is denoted by $x_i(t)$. However, $x_i(t)$ must satisfy the conditions expressed by Equations (3) and (4).

$$\text{If } |z_i(t) - z_i(t-1)| > d_i, \text{ then } x_i(t) = 1 \quad (3)$$

$$\text{If } |z_i(t) - z_i(t-1)| \leq d_i, \text{ then } x_i(t) = 0 \quad (4).$$

In Equations (3) and (4), $d_i$ is the threshold value that determines whether or not node i changes significantly at time t. In one embodiment, $X(t) = (x_1(t), ..., x_n(t))$ is defined as the "transition state" of system (1) at time t. From the above singular properties of DNM and conclusions (C1) and (C2), we can derive the following (D1) and (D2) transition state properties.

(D1)

If node i and node j nodes belong to the dominant group or DNM, the correlation between transition states $x_i(t+T)$ and $x_i(t)$ increases rapidly, and

$$p(x_i(t+T) = 1 | x_j(t) = \gamma) \rightarrow 1$$

$$p(x_i(t+T) = 0 | x_j(t) = \gamma) \rightarrow 0,$$

where $\gamma \in \{0,1\}$ and p is the transition probability.

(D2)

If neither node i nor node j belongs to the dominant group or DNB, then the transition state $x_i(t+T)$ is statistically independent of $x_j(t)$, and

$$p(x_i(t+T) = \gamma_i | x_j(t) = \gamma_j) = p(x_i(t+T) = \gamma_i) \rightarrow \alpha,$$

where $\gamma_i, \gamma_j \in \{0, 1\}$ and $\alpha \in (0, 1)$.

It should be noted that it can recover quickly from disturbances if the system is in the normal state, however it is sensitive to even small disturbances in the pre-disease state. Therefore, the above threshold $d_i$ must be set so that it can distinguish between "small changes" in the normal state and "large changes" in the pre-disease state. Here, if the system is in the normal state ($t = t_0$), $p(|z_k(t)|>d_k) = \alpha$ at each node k and each threshold d is set as in Equation (5) below. Each threshold d set by Equation (5) determines whether or not a major change or state transition has occurred between the previous state $z_i(t-1)$ and the state $z_i(t)$ at the time of the target of discrimination. In equation (5), $i_1$, $i_2$, ..., $i_m$ denote the m neighboring nodes linked to node i.

[equation 1]

$$p\left(\left|z_i(t_0)\right| > d_i, \left|z_{i_1}(t_0)\right| > d_{i_1}, ..., \left|z_{i_m}(t_0)\right| > d_{i_m}\right) \le \alpha. \qquad (5)$$

For example, each threshold $d_i$ is set so that $\alpha = 0.5$ for perturbations in the normal state, based on samples taken during the period of the normal state.

Local Network

If node i has m nodes and links, i.e., node i has m neighbors, $i_1$, $i_2$, ...$i_m$, the network centered on node i is defined as a local network. In this case, the transition state of the local network centered at node i at time t is $X_i(t) = (X_i(t), X_{i1}(t), ..., X_{im}(t))$. For the sake of simplicity, $X_i(t)$ will be denoted by $X(t)$, omitting the "i".

The connection relationship of each node i is set based on the interaction between nodes. For example, when proteins are used as nodes, information recorded in a database such as PPI, Protein-Protein Interaction, which shows interactions between proteins, can be used. Such databases are also available from websites such as BioGrid, TRED, KEGG, HPRD, etc. When proteins are used as nodes, adjacent nodes and local networks based on adjacent nodes as well as the overall network are set up based on databases such as PPI that show protein-protein interactions. In the case of using other factors as nodes, databases for the relevant factor will be used.

With regard to the transition state X(t) at the time t, there exist $2^{m+1}$ possible transition states at the next time t+1. Each of these possible transition states is a stochastic event and can be expressed by the following equation (6) using $\{A_u\}_{u1,2,...}^{m+1}$.

$$A_u = \{x_i = \gamma_0, x_{i1} = \gamma_1, ..., x_{im} = \gamma_m\} \qquad (6),$$

where $\gamma_1 \in \{0,1\}$ and $l \in \{0,1,2,...,m\}$.

Therefore, the discrete stochastic process in the local network is the following equation (7).

$$\{X(t+i)\}_{i=0,1,...} = \{X(t), X(t+1), ..., X(t+i), ...\} \qquad (7),$$

,where $X(t+i) = A_u$ and $u \in \{1, 2, ..., 2^{m+1}\}$.

In other words, when system (1) is in the normal state or the pre-disease state, the discrete stochastic process in question is a Markov process and can be defined by the Markov matrix $P = (p_{u,v})$. The transition rate from state u to state v can then be expressed by the following equation (8).

[equation 2]

$$p_{u,v}(t) = \Pr(X(t+1) = A_v \mid X(t) = A_u) \qquad (8)$$

$$\sum_v p_{u,v}(t) = 1$$

WHERE, $u, v \in \{1, 2, ..., 2^{m+1}\}$, $\sum_v p_{u,v}(t)=1$ , Pr: DISCRETE PROBABILITY.

Network flow entropy

Assume that the state transition matrix of the local network described above is stationary and unchanging for a certain period of time. $p_{u,v}(t)$ is the element in the u-th row v-th column in the state transition matrix and is the transition probability between any two possible states $A_u$ and $A_v$. Thus, for a specific period of time, normal or pre-disease, the stochastic process shown in Equation (9) below is a stationary Markov process.
[equation 3]

$$\text{STOCHASTIC PROCESS} \left\{ X(t) \right\}_{t \in [t1,\ t2]} \qquad (9)$$

Then, there exists a stationary distribution $\pi = (\pi_1, ..., \pi_2{}^{m+1})$ satisfying the following equation (10).
[equation 4]

$$\sum_v \pi_v p_{u,v} = \pi_u \qquad (10)$$

Based on the above considerations, the network flow entropy $NFE_T(x_{ik})$ is defined by the following equation (11).
[equation 5]

$$NFE_T\left(x_{i_k}\right) = -\sum_{j=1}^{n+1} x_{i_k j}\, p\left(x_{i_k j}\right) \log\left(x_{i_k j} p\left(x_{i_k j}\right)\right) \qquad (11)$$

In Equation (11), the index "i" indicates the central node i of the local network, and X indicates the state transition process X(t), X(t+1), ..., X(t+T) of the local network. Network flow entropy, shown as Equation (11), is an extension of the statistical mechanics concept of microscopic entropy.

The first embodiment

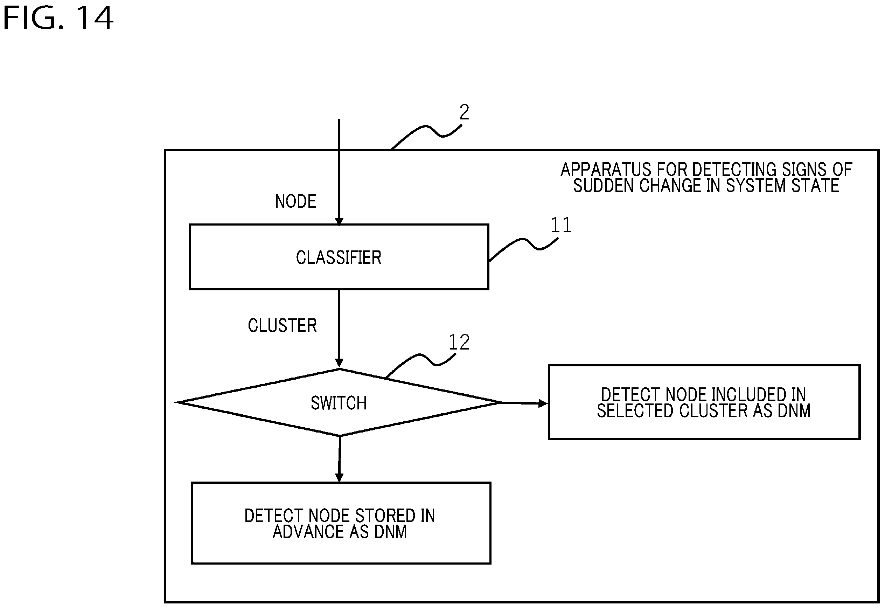

Figure 14 shows a functional block diagram of an apparatus for detecting signs of a sudden change in system state 2 according to the first embodiment. The apparatus for detecting signs of a sudden change in system state 2 detects signs of a sudden change in the state of the target system. The apparatus for detecting signs of a sudden change in system state 2 is comprises a classifier 11 and a switch 12.
The classifier 11 classifies a plurality of nodes into a plurality of clusters based on the correlation of time-series changes of measurement data concerning a plurality of nodes constituting the target system.
The switch 12 selects a cluster that satisfies predefined selection conditions based on the time-series changes of measurement data of the nodes in each classified cluster and the correlation of time-series changes of measurement data among all the nodes and switches between detecting a node in the selected cluster as the dynamical network marker that can be used as predictors of sudden state changes or detecting a specific node that has been stored in advance as the dynamical network marker.
According to the embodiment, it is possible to provide an apparatus that makes it possible to avoid the enormous amount of calculation required to detect signs of a sudden change in the state of the target system.

The second embodiment

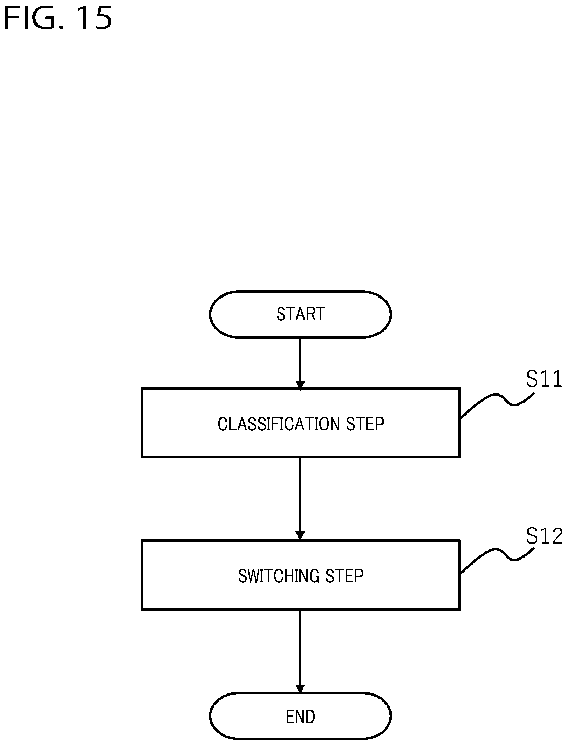

Figure 15 shows a flowchart of a method for detecting signs of a sudden change in system state according to the second embodiment. This method detects signs of a sudden change in the state of the target system.
The method comprises a classification step S11 to classify a plurality of nodes into a plurality of clusters based on the correlation of time-series changes of measurement data concerning a plurality of nodes constituting the target system and a switching step S12 to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.
According to the embodiment, it is possible to avoid the enormous amount of calculation required to detect signs of a sudden change in the state of the target system.

The third embodiment

The third embodiment is a program for detecting signs of a sudden change in system state. The program causes a computer to perform a classification step S11 to classify a plurality of nodes into a plurality of clusters based on the correlation of time-series changes of measurement data concerning a plurality of nodes constituting the target system and a switching step S12 to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

According to the embodiment, it is possible to implement in software a program that makes it possible to avoid the enormous amount of calculation required to detect signs of a sudden change in the state of the target system.

The fourth embodiment

The fourth embodiment is a recording medium. This recording medium records a program which causes a computer to perform a classification step S11 to classify a plurality of nodes into a plurality of clusters based on the correlation of time-series changes of measurement data concerning a plurality of nodes constituting the target system and a switching step S12 to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

According to the embodiment, it is possible to record a program on a recording medium that makes it possible to avoid the enormous amount of calculation required to detect signs of a sudden change in the state of the target system.

The fifth embodiment

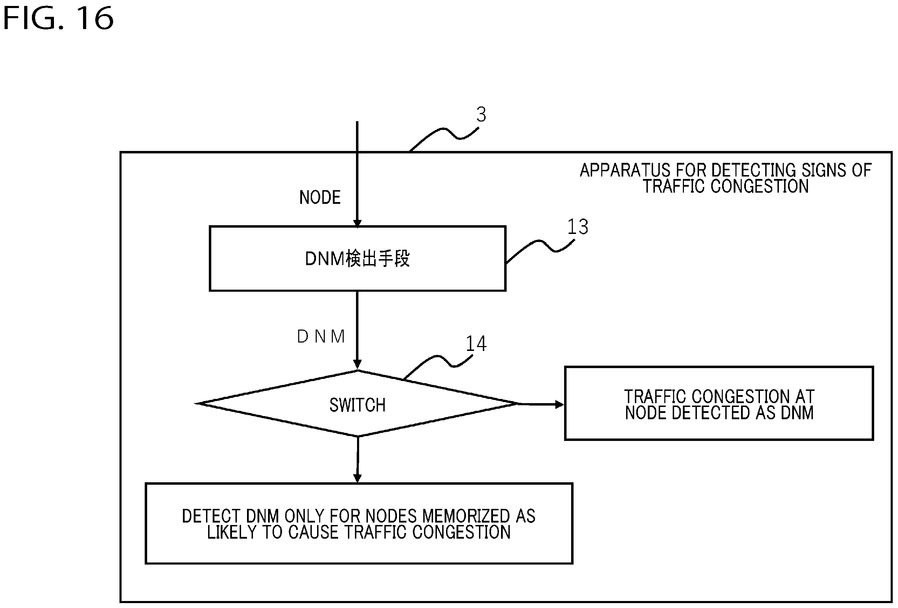

Figure 16 shows a functional block diagram of an apparatus for detecting signs of traffic congestion 3 according to the fifth embodiment. The apparatus for detecting signs of traffic congestion 3 detects signs of traffic congestion on the road. The device 3 comprises a dynamical network marker, DNM, detector 13 and a switch 14.

The dynamical network marker detector 13 detects a dynamical network marker based on the dispersion of traffic volume at each node and the correlation of traffic volume among the nodes, where multiple points on the road are nodes.

The switch 14 switches between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

According to the embodiment, it is possible to provide an apparatus that makes it possible to avoid the enormous amount of calculation required to detect signs of a sudden change in traffic congestion on the road.

The sixth embodiment

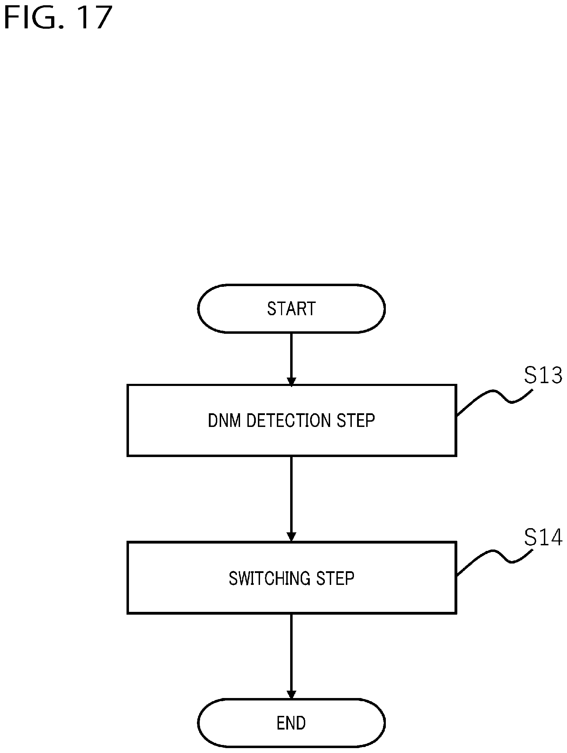

Figure 17 shows a flowchart of a method for detecting signs of traffic congestion according to the sixth embodiment. This method detects signs of traffic congestion on roads.

This method comprises a dynamical network marker (DNM) detection step S13, which detects the dynamical network marker based on the dispersion of traffic volume at each node and the correlation of traffic volume among the nodes, where multiple points on the road are nodes and a switching step S14, which switches between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

According to this method, it is possible to avoid the enormous amount of calculation required to detect signs of a sudden change in traffic congestion on the road.

The seventh embodiment

The seventh embodiment is a program for detecting signs of traffic congestion. This program causes a computer to perform a dynamical network marker (DNM) detection step S13, which detects the dynamical network marker based on the dispersion of traffic volume at each node and the correlation of traffic volume among the nodes, where multiple points on the road are nodes and a switching step S14, which switches between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

According to this method, it is possible to implement in software a program that makes it possible to avoid the enormous amount of calculation required to detect signs of a sudden change in traffic congestion on the road.

The eighth embodiment

The eighth embodiment is a recording medium. This recording medium records a program which causes a computer to perform a dynamical network marker (DNM) detection step S13, which detects the dynamical network marker based on the dispersion of traffic volume at each node and the correlation of traffic volume among the nodes, where multiple points on the road are nodes and a switching step S14, which switches between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

According to the embodiment, it is possible to record a program on a recording medium that makes it possible to avoid the enormous amount of calculation required to detect signs of a sudden change in traffic congestion on the road.

The ninth embodiment

Figure 16 shows a functional block diagram of an apparatus for detecting pre-disease state 1 according to the ninth embodiment. The apparatus for detecting pre-disease state 1 is a device that detects the pre-disease state which is the turning point from the normal state to the disease state. The apparatus for detecting pre-disease state 1 comprises a data generator 10, a sample network calculator 20, a sample specific network calculator 30, a sample specific differential network calculator 40, a local network extractor 50, a gene expression probability calculator 60, a network flow entropy calculator 70, a differential network flow entropy calculator 80 and a temporal differential network flow entropy calculator 90.

The data generator 10 generates a reference gene expression data generated from biological samples collected from a population including multiple persons and a gene expression data for testing, which is the gene expression data of biological samples collected from subjects at a predetermined time added to this reference gene expression data.

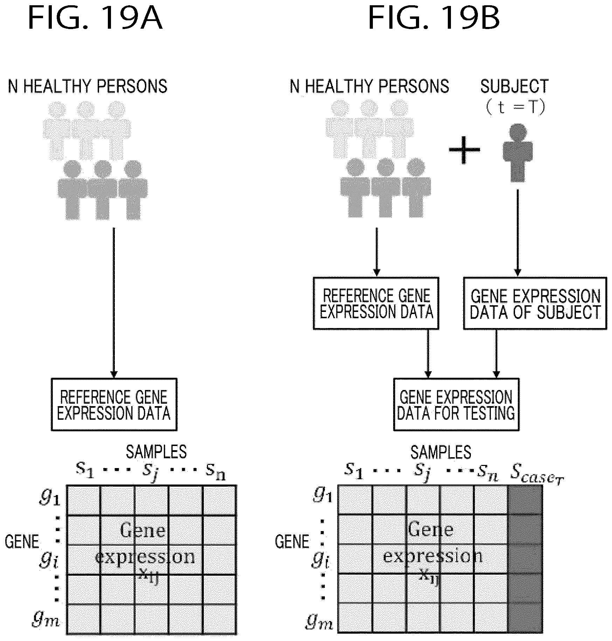

Figure 19 schematically shows the operation of the data generator 10. Figure 19 a shows the generation of reference gene expression data from biological samples taken from a population of n healthy persons. Here, these biological samples are assumed to be related to m different genes. Figure 19 b shows how the gene expression data for testing is generated by adding the gene expression data from the biological samples collected from the subjects at time t=T to this reference gene expression data. Gene expression data is data representing information on gene expression levels obtained from biological samples such as blood. Highthroughput technology, for example, may be used to measure genes from biological samples.

In this example, the reference gene expression data is an m-by-n matrix in which the (i, j) components represent the expression levels of the i-th (i=1, ..., m) gene in the j-th (j=1, ..., n) person. The gene expression data for the test is an m-by-n+1 matrix in which the (i, j) component represents the expression level of the i-th (i=1, ..., m) gene of the j-th (j=1, ..., n) person and the (i, n+1) component represents the expression level of the i-th (i=1, ..., m) gene of the subject.

The sample network calculator 20 calculates a reference sample network from the reference gene expression data and a perturbation sample network from the gene expression data for testing.

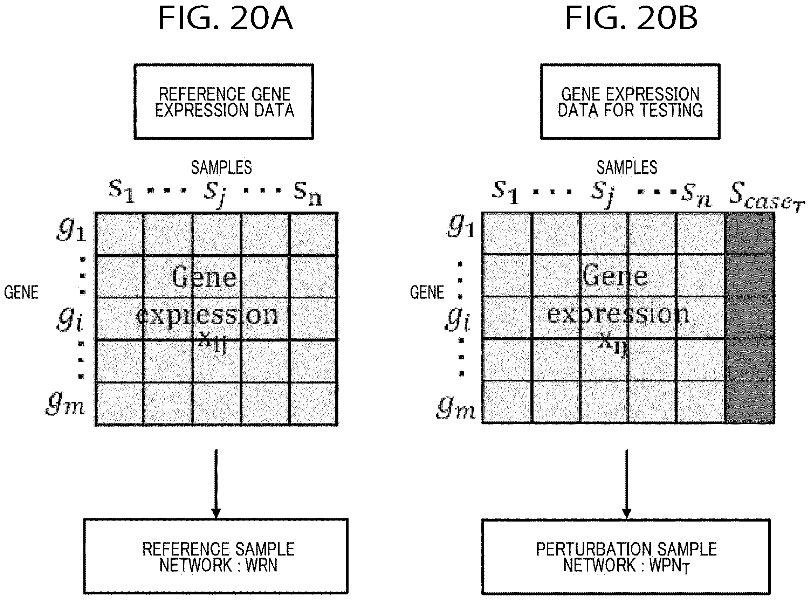

Figure 20 schematically shows the operation of the sample network calculator 20. Figure 20 a shows the calculation of the reference sample network from the reference gene expression data. Fig. 20 b shows the calculation of the perturbation sample network at time t=T from the gene expression data for testing.

In this example, the reference sample network is a Weighted Reference Network (WRN) generated from the reference gene expression data and the perturbation sample network is a Weighted Perturbation Network ($WPN_T$) generated from the gene expression data for testing.

The sample specific network calculator 30 calculates a sample specific network. Here, the sample specific network is a network that represents the relationship between the reference sample network and the perturbation sample network.

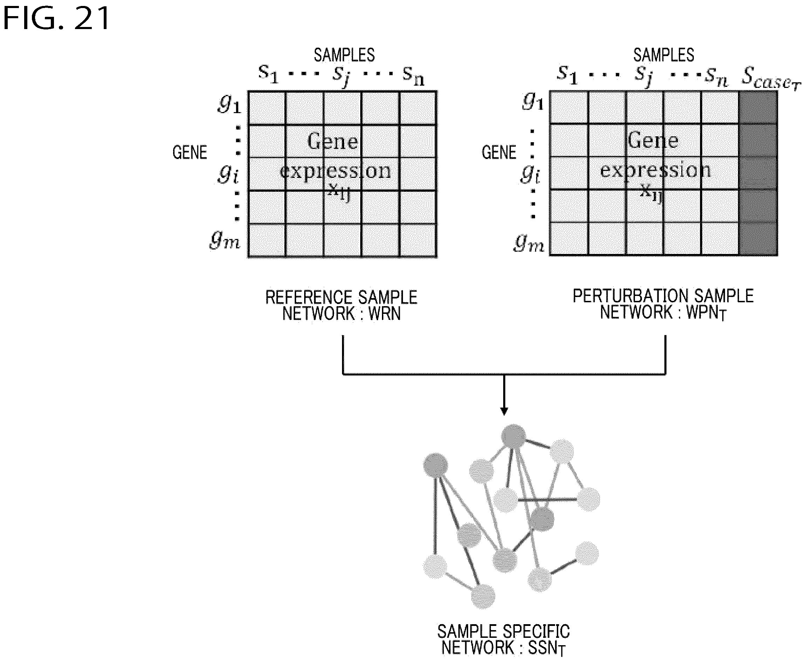

Figure 21 schematically shows the operation of the sample specific network calculator 30. The sample specific network calculator 30 generates a sample specific network ($SSN_T$) at time t=T by comparing the reference sample network (WRN) and the perturbation sample network ($WPN_T$) at time t=T.

In this example, the sample specific network ($SSN_T$) at time t=T is calculated by, for example, comparing the reference sample network (WRN) with the perturbation sample network ($WPN_T$) at time t=T and leaving only the different edge. Specifically, the network indicated by $|WPN_T\text{-}WRN|$ is calculated, and the result which remains as the different edge may be the sample specific network ($SSN_T$) at time t=T.

The sample specific differential network calculator 40 calculates a sample specific differential network from the sample specific network at the first time and the sample specific network at the second time after the first time.

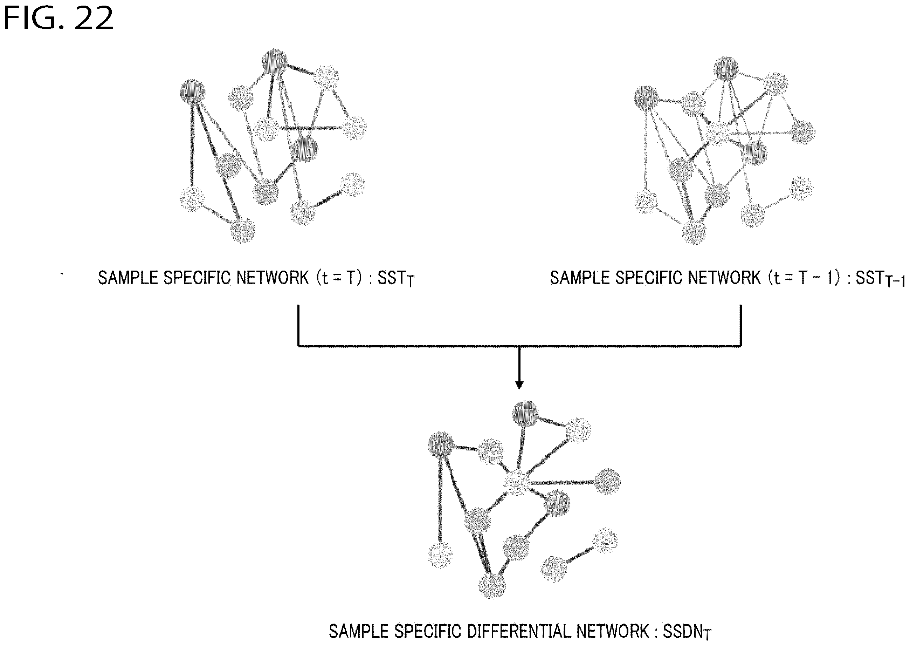

Figure 22 schematically shows the operation of the sample specific differential network calculator 40. In this example, the sample specific differential network (SSDN$_T$) at t=T compares the sample specific network (SSN$_T$) at t=T-1 (first time) and the sample specific network (SSN$_{T-1}$) at t=T (second time) and leaving only the different edge.

The local network extractor 50 extracts a local network. Here, the local network is a network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene.

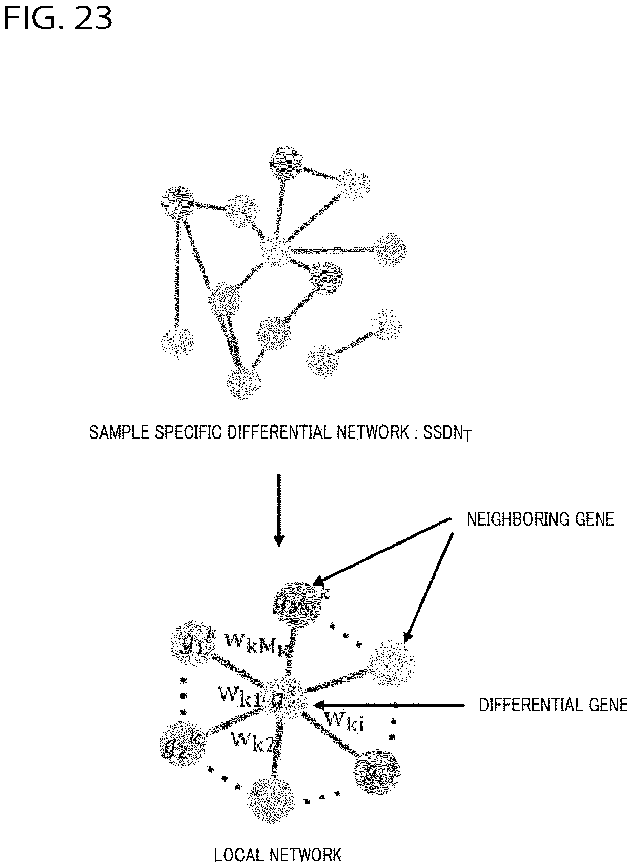

Figure 23 shows schematically shows the operation of the local network extractor 50. Each of the local networks $N^k$ (k=1, ..., l) is calculated from the sample specific network (SSN$_{T-1}$). However, l is the number of genes. A differential gene $g^k$ is placed in the center of the local network, and neighboring genes $g_i^k$ (i=1,..., M$_k$) are placed around it. The node topological dissimilarity $W_{ik}$ represents the strength of interaction between the differential gene $g^k$ at the center and the surrounding neighboring genes $g_i^k$.

The gene expression probability calculator 60 calculates a gene expression probability, which is the probability that each neighboring gene is expressed.

The gene expression probability may be calculated assuming that the expression of each adjacent gene follows a normal distribution.

For example, the expression probability of gene $x_{ikj}$ in the mixed sample {$s_1, ... , s_n, s_{caseT}$} may be expressed by the probability distribution function $f(x_{ikj})$ in Equation (12) assuming that it follows a normal distribution.

[equation 6]

$$f\left(x_{i_k j}\right) = \frac{1}{\sqrt{2\pi\sigma^2}} e^{\frac{-(x_{i_k j}-\mu_{i_k})^2}{2(\sigma_{i_k})^2}} \qquad (1\,2)$$

Here, $x_{ikj}$ represents the (ik, j) component of the expression data of the i-th gene $g_i^k$ in the k-th local network. $\mu_{ik}$ and $\sigma_{ik}$ are the mean deviation and the standard deviation of gene $g_i^k$, respectively.

In this case, the cumulative distribution function $F(x_{ikj})$ is expressed in Equation (13).

[equation 7]

$$F\left(x_{i_k j}\right) = \frac{1}{\sqrt{2\pi\sigma^2}} \int_{-\infty}^{x_{i_k j}} e^{\frac{-(t-\mu_{i_k})^2}{2(\sigma_{i_k})^2}} dt \qquad (1\,3)$$

In this case, the gene expression probability $p(x_{ikj})$ is calculated as follows.

[equation 8]

$$p\left(x_{i_k j}\right) = \frac{\frac{1}{\sqrt{2\pi\sigma^2}} \int_{-\infty}^{x_{i_k j}} e^{\frac{-\left(t-\mu_{i_k}\right)^2}{2\left(\sigma_{i_k}\right)^2}} dt}{\sum_{j=1}^{n+1} \frac{1}{\sqrt{2\pi\sigma^2}} \int_{-\infty}^{x_{i_k j}} e^{\frac{-\left(t-\mu_{i_k}\right)^2}{2\left(\sigma_{i_k}\right)^2}} dt} \qquad (1\,4)$$

The network flow entropy calculator 70 calculates a local network flow entropy and a conditional network flow entropy under the condition that the differential gene expression has a given value, for each neighboring gene based on the gene expression probability.

For example, the network flow entropy calculator 70 may calculate the local network flow entropy and the conditional network flow entropy with respect to the neighboring gene $g_i^k$ in the local network $N^k$ (k=1, ..., l). Here, $x_{ik}$ is the expression data of the i-th gene $g_i^k$ in the k-th local network and $x_k$ is the expression data across genes in the k-th local network. In this case, the local network flow entropy NFE$_T(x_{ik})$ is calculated as follows.

[equation 5]

$$NFE_T\left(x_{i_k}\right) = -\sum_{j=1}^{n+1} x_{i_k j}\, p\left(x_{i_k j}\right) \log\left(x_{i_k j} p\left(x_{i_k j}\right)\right) \qquad (11)$$

The conditional network flow entropy $NFE_T$ $(x_{ik/}x_k)$ is calculated as follows.
[equation 9]

$$NFE_T\left(x_{i_k}/x_k\right) = -\sum_{j=1}^{n+1} x_{i_k j}\, p\left(x_{kj}, x_{i_k j}\right) \log\left(x_{i_k j} p\left(x_{i_k j}/x_{kj}\right)\right) \qquad (15)$$

Here, the following equations hold.
[equation 10]

$$p\left(x_{kj}, x_{i_k j}\right) = p\left(x_{kj}\right) p\left(x_{i_k j}/x_{kj}\right) \qquad (16)$$

[equation 11]

$$p\left(x_{i_k j}/x_{kj}\right) = \frac{W_{ik}}{\sum_{i=1}^{M_k} W_{ik}} \qquad (17)$$

The differential network flow entropy calculator 80 calculates a differential network flow entropy from the local network flow entropy and the conditional network flow entropy.

The differential network flow entropy DNFE can be calculated, for example, as follows. The differential network flow entropy at time t=T is defined as the difference between the local network flow entropy and the conditional network flow entropy, i.e., $NFE_T$ $(x_{ik})$ - $NFE_T$ $(x_{ik/}x_k)$. This can be considered to be the difference between the local network flow entropy and the conditional network flow entropy of the adjacent gene $g_i^k$. By averaging the differential network flow entropy for all adjacent genes, the differential network flow entropy $DNFE_T^k$ can be calculated as follows.
[equation 12]

$$DNFE_T{}^k = \frac{\sum_{i=1}^{M_k}\left(NFE_T\left(x_{i_k}\right) - NFE_T\left(x_{i_k}/x_k\right)\right)}{M_k} \qquad (18)$$

Here, $M_k$ is the number of adjacent genes.

The differential network flow entropy $DNFE_T^k$ can be interpreted as the mutual flow information between the differential gene $g^k$ and its neighbor gene $g_i^k$ in the local network $N^k$ (k=1, ..., l) at time t=T.

The temporal differential network flow entropy calculator 90 calculates a temporal differential network flow entropy from the differential network flow entropy.

The temporal differential network flow entropy $TNFE_T$ at time t=T can be calculated by averaging the mutual flow information of all local networks in the sample specific differential network ($SSDN_T$) as follows.
[equation 13]

$$TNFE_T = \frac{\sum_{k=1}^{l} DNFE_T{}^k}{l} \qquad (19)$$

Here, l is the number of local networks.

T can be considered to be a tipping point of state transition if the temporal differential network flow entropy $TNFE_T$ at time t = T changes abruptly. In other words, the change in temporal differential network flow entropy can detect the pre-disease state.

The pre-disease state detector 100 detects a pre-disease state when the difference between the temporal differential network flow entropy at the first time and the temporal differential network flow entropy at the second time is more than a predetermined value.

For example, the pre-disease state detector 100 may detect a pre-disease state when the temporal differential network flow entropy at the second time is twice or more than the temporal differential network flow entropy at the first time.

Using network flow entropy as a measure for detecting DNM improves noise tolerance compared to using conventional Pearson correlation coefficient or local network entropy. In addition, biological samples can be collected only twice, once from a population including multiple persons and once from the examinee at a given time. Therefore, according to the embodiment, it is possible to detect a pre-disease state by realizing DNM detection with high noise tolerance and a small number of biological material collections from the subject.

The following are examples of the embodiment explained above.

Example 1

The data generator 10 generates reference gene expression data that is an m-by-n matrix such that the (i, j) component represents the expression level of the i-th (i=1, ..., m) gene in the j-th (j=1, ..., n) person, where the number of the persons is n and the number of gene types is m. The data generator 10 generates the gene expression data for testing that is an m-by-n+1 matrix such that the (i, j) component represents the expression level of the i-th (i=1, ..., m) gene of the j-th (j=1, ..., n) person and the (i, n+1) component represents the expression level of the i-th (i=1, ..., m) gene of the subject.

According to this example, it is possible to generate reference gene expression data and gene expression data for testing in matrix form.

Example 2

The sample network calculator 20 calculates the reference sample network as a weighted gene expression network generated from the reference gene expression data and the perturbation sample network as a weighted gene expression network generated from the gene expression data for testing.

According to this example, the reference sample network and perturbation sample network can be calculated accurately.

Example 3

The sample specific network calculator 30 calculates the sample specific network by comparing the reference sample network and the perturbation sample network and leaving different edges.

According to this example, the sample specific network can be calculated accurately.

Example 4

The sample specific differential network calculator 40 compares the sample specific network at the first time with the sample specific network at the second time, and calculates the sample specific differential network by leaving different edges.

According to this example, the sample specific differential network can be calculated accurately.

Example 5

The gene expression probability calculator 60 calculates the gene expression probability assuming that the expression of each neighboring gene follows a normal distribution.

According to this example, the gene expression probability can be calculated accurately.

Example 6

The gene expression probability calculator 60 calculates the gene expression probability $p(x_{ikj})$ to be:
[equation 8]

$$p\left(x_{i_k j}\right) = \frac{\dfrac{1}{\sqrt{2\pi\sigma^2}} \int_{-\infty}^{x_{i_k j}} e^{\dfrac{-\left(t-\mu_{i_k}\right)^2}{2\left(\sigma_{i_k}\right)^2}} dt}{\sum_{j=1}^{n+1} \dfrac{1}{\sqrt{2\pi\sigma^2}} \int_{-\infty}^{x_{i_k j}} e^{\dfrac{-\left(t-\mu_{i_k}\right)^2}{2\left(\sigma_{i_k}\right)^2}} dt} \qquad (14)$$

, where the (ik, j) component of the expression data of the i-th gene $g_i^k$ in the k-th local network is $x_{ikj}$, and $\mu_{ik}$ and $\sigma_{ik}$ are the mean deviation and the standard deviation of the gene $g_i^k$, respectively,

According to this example, the gene expression probability can be calculated more accurately.

Example 7

The network flow entropy calculator 70 calculates the local network flow entropy $NFE_T(x_{ik})$ to be:
[equation 5]

$$NFE_T\left(x_{i_k}\right) = -\sum_{j=1}^{n+1} x_{i_k j}\, p\left(x_{i_k j}\right) \log\left(x_{i_k j} p\left(x_{i_k j}\right)\right) \qquad (11)$$

and the conditional network flow entropy $NFE_T (x_{ik}/x_k)$ to be [equation 9]

$$NFE_T\left(x_{i_k}/x_k\right) = -\sum_{j=1}^{n+1} x_{i_k j}\, p\left(x_{kj}, x_{i_k j}\right) \log\left(x_{i_k j} p\left(x_{i_k j}/x_{kj}\right)\right) \qquad (15)$$

, where the expression data of the i-th gene $g_i^k$ in the k-th local network is $x_{ik}$ and the expression data over the entire gene in the k-th local network is $x_k$.

According to this example, the local network flow entropy and the conditional network flow entropy can be calculated more accurately.

Example 8

The differential network flow entropy calculator 80 calculates the differential network flow entropy $DNFE_T^k$ to be:
[equation 12]

$$DNFE_T{}^k = \frac{\sum_{i=1}^{M_k} \left(NFE_T\left(x_{i_k}\right) - NFE_T\left(x_{i_k}/x_k\right)\right)}{M_k} \qquad (18)$$

, where the number of adjacent genes is $M_k$.

According to this example, the differential network flow entropy can be calculated accurately.

Example 9

The temporal differential network flow entropy calculator 90 calculates the temporal differential network flow entropy to be:
[equation 13]

$$TNFE_T = \frac{\sum_{k=1}^{l} DNFE_T{}^k}{l} \qquad (19)$$

, where the number of local networks is l.

According to this example, the temporal differential network flow entropy can be calculated accurately.

Example 10

The pre-disease state detector 100 detects a pre-disease state when the temporal differential network flow entropy at the second time is twice or more than the temporal differential network flow entropy at the first time.

According to this example, the pre-disease state can be detected more accurately.

The tenth embodiment

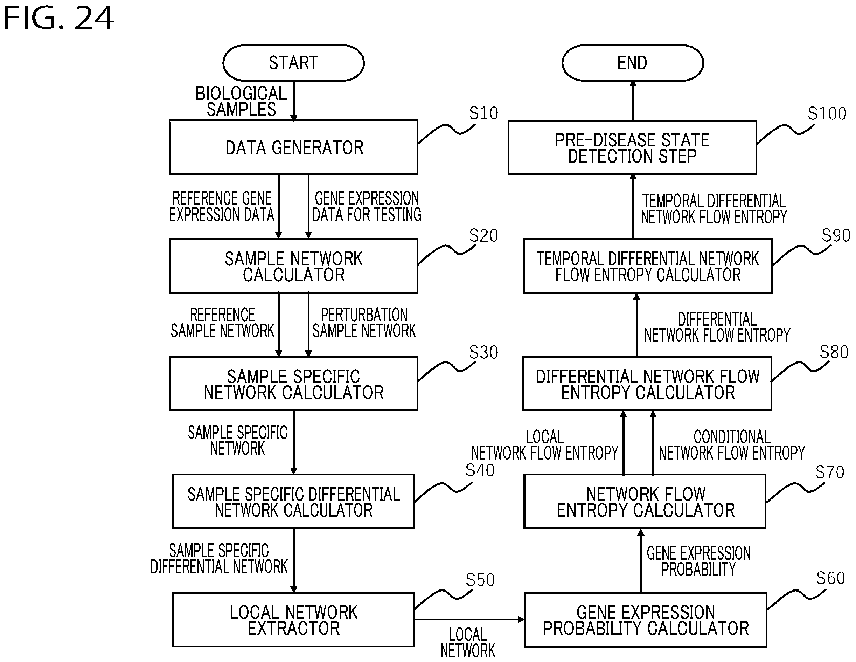

Figure 24 shows a flowchart of a method for detecting pre-disease state according to the tenth embodiment. This method detects a pre-disease state which is a state that transitions from a normal state to a disease state. The method for detecting a pre-disease state comprises a data generation step S10, a sample network calculation step S20, a sample specific network calculation step S30, a sample specific differential network calculation step S40, a local network extraction step S50, a gene expression probability calculation step S60, a network flow entropy calculation step S70, a differential network flow entropy calculation step S80 and a temporal differential network flow entropy calculation step S90.

The data generation step S10 generates a reference gene expression data generated from biological samples collected from a population including multiple persons and a gene expression data for testing, which is the gene expression data of biological samples collected from subjects at a predetermined time added to this reference gene expression data.

The sample network calculation step S20 calculates a reference sample network from the reference gene expression data and a perturbation sample network from the gene expression data for testing.

The sample specific network calculation step S30 calculates a sample specific network. Here, the sample specific network is a network that represents the relationship between the reference sample network and the perturbation sample network.

The sample specific differential network calculation step S40 calculates a sample specific differential network from the sample specific network at the first time and the sample specific network at the second time after the first time.

Local network extraction step S50 extracts a local network. Here, the local network is a network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene.

The gene expression probability calculation step S60 calculates a gene expression probability, which is the probability that each neighboring gene is expressed.

The network flow entropy calculation step S70 calculates a local network flow entropy and a conditional network flow entropy under the condition that the differential gene expression has a given value, for each neighboring gene based on the gene expression probability.

The differential network flow entropy calculation step S80 calculates a differential network flow entropy from the local network flow entropy and the conditional network flow entropy.

The temporal differential network flow entropy calculation step S90 calculates a temporal differential network flow entropy from the differential network flow entropy.

The pre-disease state detection step S100 detects a pre-disease state when the difference between the temporal differential network flow entropy at the first time and the temporal differential network flow entropy at the second time is more than a predetermined value.

According to the embodiment, it is possible to detect a pre-disease state by realizing DNM detection with high noise tolerance and a small number of biological material collections from the subject.

The eleventh embodiment

The eleventh embodiment is a program. This program causes a computer to perform a method for detecting pre-disease state which comprises a data generation step S10, a sample network calculation step S20, a sample specific network calculation step S30, a sample specific differential network calculation step S40, a local network extraction step S50, a gene expression probability calculation step S60, a network flow entropy calculation step S70, a differential network flow entropy calculation step S80 and a temporal differential network flow entropy calculation step S90.

The data generation step S10 generates a reference gene expression data generated from biological samples collected from a population including multiple persons and a gene expression data for testing, which is the gene expression data of biological samples collected from subjects at a predetermined time added to this reference gene expression data.

The sample network calculation step S20 calculates a reference sample network from the reference gene expression data and a perturbation sample network from the gene expression data for testing.

The sample specific network calculation step S30 calculates a sample specific network. Here, the sample specific network is a network that represents the relationship between the reference sample network and the perturbation sample network.

The sample specific differential network calculation step S40 calculates a sample specific differential network from the sample specific network at the first time and the sample specific network at the second time after the first time.

Local network extraction step S50 extracts a local network. Here, the local network is a network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene.

The gene expression probability calculation step S60 calculates a gene expression probability, which is the probability that each neighboring gene is expressed.

The network flow entropy calculation step S70 calculates a local network flow entropy and a conditional network flow entropy under the condition that the differential gene expression has a given value, for each neighboring gene based on the gene expression probability.

The differential network flow entropy calculation step S80 calculates a differential network flow entropy from the local network flow entropy and the conditional network flow entropy.

The temporal differential network flow entropy calculation step S90 calculates a temporal differential network flow entropy from the differential network flow entropy.

The pre-disease state detection step S100 detects a pre-disease state when the difference between the temporal differential network flow entropy at the first time and the temporal differential network flow entropy at the second time is more than a predetermined value.

According to the embodiment, it is possible to implement in software a program that makes it possible to detect a pre-disease state by realizing DNM detection with high noise tolerance and a small number of biological material collections from the subject.

The twelfth embodiment

The twelfth embodiment is a recording medium. This recording medium records a program which causes a computer to perform a method for detecting pre-disease state which comprises a data generation step S10, a sample network calculation step S20, a sample specific network calculation step S30, a sample specific differential network calculation step S40, a local network extraction step S50, a gene expression probability calculation step S60, a network flow entropy calculation step S70, a differential network flow entropy calculation step S80 and a temporal differential network flow entropy calculation step S90.

The data generation step S10 generates a reference gene expression data generated from biological samples collected from a population including multiple persons and a gene expression data for testing, which is the gene expression data of biological samples collected from subjects at a predetermined time added to this reference gene expression data.

The sample network calculation step S20 calculates a reference sample network from the reference gene expression data and a perturbation sample network from the gene expression data for testing.

The sample specific network calculation step S30 calculates a sample specific network. Here, the sample specific network is a network that represents the relationship between the reference sample network and the perturbation sample network.

The sample specific differential network calculation step S40 calculates a sample specific differential network from the sample specific network at the first time and the sample specific network at the second time after the first time.

Local network extraction step S50 extracts a local network. Here, the local network is a network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene.

The gene expression probability calculation step S60 calculates a gene expression probability, which is the probability that each neighboring gene is expressed.

The network flow entropy calculation step S70 calculates a local network flow entropy and a conditional network flow entropy under the condition that the differential gene expression has a given value, for each neighboring gene based on the gene expression probability.

The differential network flow entropy calculation step S80 calculates a differential network flow entropy from the local network flow entropy and the conditional network flow entropy.

The temporal differential network flow entropy calculation step S90 calculates a temporal differential network flow entropy from the differential network flow entropy.

The pre-disease state detection step S100 detects a pre-disease state when the difference between the temporal differential network flow entropy at the first time and the temporal differential network flow entropy at the second time is more than a predetermined value.

According to the embodiment, it is possible to record a program on a recording medium that makes it possible to detect a pre-disease state by realizing DNM detection with high noise tolerance and a small number of biological material collections from the subject.

The thirteenth embodiment

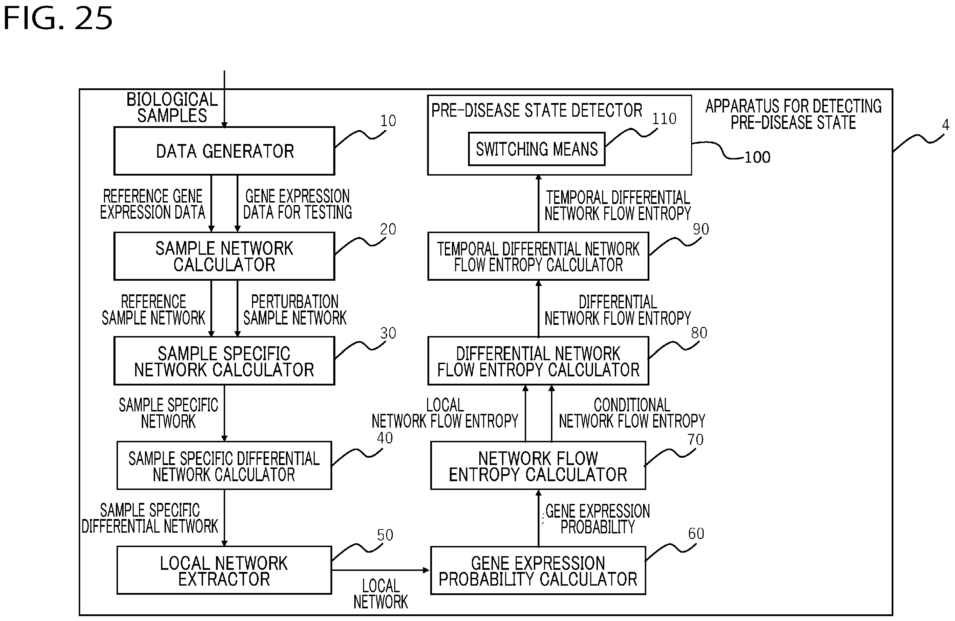

Figure 25 shows a functional block diagram of an apparatus for detecting pre-disease state 4 according to the thirteenth embodiment. The apparatus for detecting pre-disease state 4 comprises a data generator 10, a sample network calculator 20, a sample specific network calculator 30, sample specific differential network calculator 40, a local network extractor 50, a gene expression probability calculator 60, a network flow entropy calculator 70, a differential network flow entropy calculator 80, a temporal differential network flow entropy calculator 90, a pre-disease state detector 100. The data generator 10 generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data. The sample network calculator 20 calculates a reference sample network from the reference gene expression data and a perturbation sample network from the gene expression data for testing. The sample specific network calculator 30 calculates a sample specific network which represents the relationship between the reference sample network and the perturbation sample network. The sample specific differential network calculator 40 calculates a sample specific differential network from the sample specific network at the first time and the sample specific network at the second time after the first time. The local network extractor 50 extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network. The gene expression probability calculator 60 calculates a gene expression probability, which is the probability that each neighboring gene is expressed. The network flow entropy calculator 70 calculates a local network flow entropy and a conditional network flow entropy under the condition that the differential gene expression has a given value, for each neighboring gene based on the gene expression probability. The differential network flow entropy calculator 80 calculates a differential network flow entropy from the local network flow entropy and the conditional network flow entropy. The temporal differential network flow entropy calculator 90 calculates a temporal differential network flow entropy from the differential network flow entropy.

The pre-disease state detector 100 comprises a switching means 110. The switching means 110 switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

According to the embodiment, it is possible provide an apparatus to detect a pre-disease state by realizing DNM detection with high noise tolerance and a small number of biological material collections from the subject.

The fourteenth embodiment

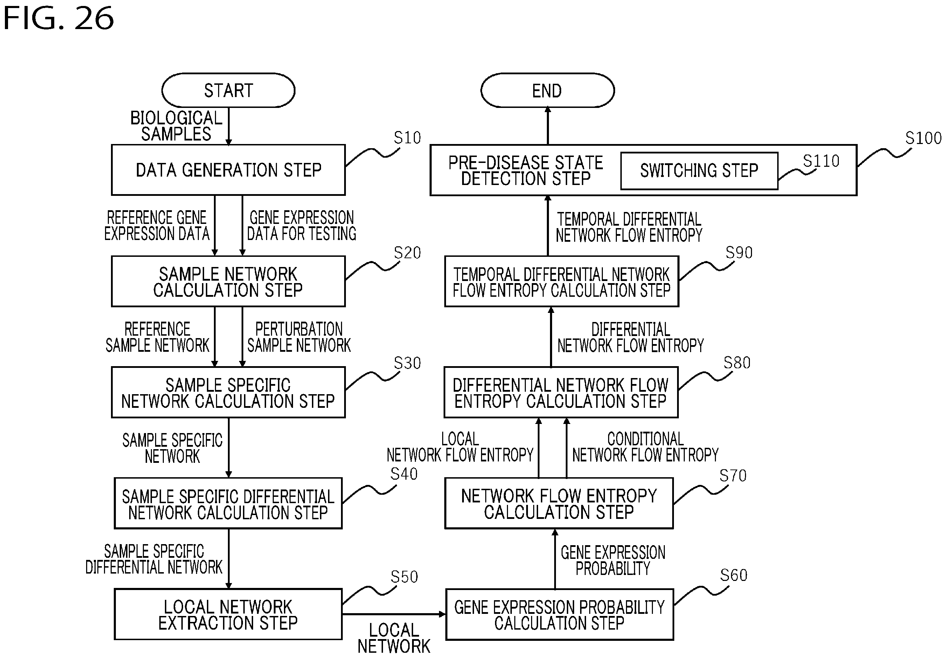

Figure 26 shows a flowchart of a method for detecting pre-disease state according to the fourteenth embodiment of. This method detects a pre-disease state which is a state that transitions from a normal state to a disease state. The method for detecting a pre-disease state comprises a data generation step S10, a sample network calculation step S20, a sample specific network calculation step S30, sample specific differential network calculation step S40, a local network extraction step S50, a gene expression probability calculation step S60, a network flow entropy calculation step S70, a differential network flow entropy calculation step S80, a temporal differential network flow entropy calculation step S90, a pre-disease state detection step S100. The data generation step S10 generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data. The sample network calculation step S20 calculates a reference sample network from the reference gene expression data and a perturbation sample network from the gene expression data for testing. The sample specific network calculation step S30 calculates a sample specific network which represents the relationship between the reference sample network and the perturbation sample network. The sample specific differential network calculation step S40 calculates a sample specific differential network from the sample specific network at the first time and the sample specific network at the second time after the first time. The local network extraction step S50 extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network. The gene expression probability calculation step S60 calculates a gene expression probability, which is the probability that each neighboring gene is expressed. The network flow entropy calculation step S70 calculates a local network flow entropy and a conditional network flow entropy under the condition that the differential gene expression has a given value, for each neighboring gene based on the gene expression probability. The differential network flow entropy calculation step S80 calculates a differential network flow entropy from the local network flow entropy and the conditional network flow entropy. The temporal differential network flow entropy calculation step S90 calculates a temporal differential network flow entropy from the differential network flow entropy.

The pre-disease state detection step comprises a switching step S110. The switching step S110 switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

According to the embodiment, it is possible to detect a pre-disease state by realizing DNM detection with high noise tolerance and a small number of biological material collections from the subject.

The fifteenth embodiment

The fifteenth embodiment is a program. This program causes a computer to perform a method for detecting pre-disease state which comprises a data generation step S10, a sample network calculation step S20, a sample specific network calculation step S30, sample specific differential network calculation step S40, a local network extraction step S50, a gene expression probability calculation step S60, a network flow entropy calculation step S70, a differential network flow entropy calculation step S80, a temporal differential network flow entropy calculation step S90, a pre-disease state detection step S100. The data generation step S10 generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data. The sample network calculation step S20 calculates a reference sample network from the reference gene expression data and a perturbation sample network from the gene expression data for testing. The sample specific network calculation step S30 calculates a sample specific network which represents the relationship between the reference sample network and the perturbation sample network. The sample specific differential network calculation step S40 calculates a sample specific differential network from the sample specific network at the first time and the sample specific network at the second time after the first time. The local network extraction step S50 extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network. The gene expression probability calculation step S60 calculates a gene expression probability, which is the probability that each neighboring gene is expressed. The network flow entropy calculation step S70 calculates a local network flow entropy and a conditional network flow entropy under the condition that the differential gene expression has a given value, for each neighboring gene based on the gene expression probability. The differential network flow entropy calculation step S80 calculates a differential network flow entropy from the local network flow entropy and the conditional network flow entropy. The temporal differential network flow entropy calculation step S90 calculates a temporal differential network flow entropy from the differential network flow entropy.

The pre-disease state detection step comprises a switching step S110. The switching step S110 switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

According to the embodiment, it is possible to implement in software a program that makes it possible to detect a pre-disease state by realizing DNM detection with high noise tolerance and a small number of biological material collections from the subject.

The sixteenth embodiment

The sixteenth embodiment is a recording medium. This recording medium records a program which causes a computer to perform a method for detecting pre-disease state which comprises a data generation step S10, a sample network calculation step S20, a sample specific network calculation step S30, sample specific differential network calculation step S40, a local network extraction step S50, a gene expression probability calculation step S60, a network flow entropy calculation step S70, a differential network flow entropy calculation step S80, a temporal differential network flow entropy calculation step S90, a pre-disease state detection step S100. The data generation step S10 generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data. The sample network calculation step S20 calculates a reference sample network from the reference gene expression data and a perturbation sample network from the gene expression data for testing. The sample specific network calculation step S30 calculates a sample specific network which represents the relationship between the reference sample network and the perturbation sample network. The sample specific differential network calculation step S40 calculates a sample specific differential network from the sample specific network at the first time and the sample specific network at the second time after the first time. The local network extraction step S50 extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network. The gene expression probability calculation step S60 calculates a gene expression probability, which is the probability that each neighboring gene is expressed. The network flow entropy calculation step S70 calculates a local network flow entropy and a conditional network flow entropy under the condition that the differential gene expression has a given value, for each neighboring gene based on the gene expression probability. The differential network flow entropy calculation step S80 calculates a differential network flow entropy from the local network flow entropy and the conditional network flow entropy. The temporal differential network flow entropy calculation step S90 calculates a temporal differential network flow entropy from the differential network flow entropy.

The pre-disease state detection step comprises a switching step S110. The switching step S110 switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

According to the embodiment, it is possible to record a program on a recording medium that makes it possible to detect a pre-disease state by realizing DNM detection with high noise tolerance and a small number of biological material collections from the subject.

The above description is based on examples of the invention. It is understood by those skilled in the art that these examples are illustrative and that various variations are possible in the combination of their respective components and respective processing processes and that such variations are also within the scope of the invention.

Variation 1

In the embodiment, a reference gene expression data is generated from biological samples collected from a population including multiple persons. However, a reference gene expression data may also be generated from biological samples taken at multiple points in the subject's past.

Variation 2

In the embodiment, information on gene expression levels obtained from biological samples such as blood was used as gene expression data. However, it is not limited to this, and in the variant example, it is possible to take into account various factors such as the type of disease and the purpose to be detected, etc. In particular, various measurement data can be used as factors, as long as the information is obtained from measurements on the living body. For example, as mentioned above, measurement data related to genes, proteins and metabolites, or data not limited to these, can be used as measurement data by quantifying various conditions at each site based on internal body images output by a measurement device such as a CT scan, etc.

In understanding the technical ideas abstracted from the embodiments and variations, the technical ideas should not be interpreted as limited to the contents of the embodiments and variations. The aforementioned embodiments and variations are merely concrete examples, and many design changes, such as modifications, additions, and deletions of components, are possible. In the embodiments, the contents where such design changes are possible are emphasized with the notation "embodiments". However, design changes are also allowed for contents without such notation.

INDUSTRIAL APPLICABILITY

The technology of the present disclosure can be used in the fields of early detection and early treatment of diseases in the medical field, development of new drugs, development of medical devices, etc.

REFERENCE SIGNS LIST

1 Apparatus for detecting pre-disease state,
2 Apparatus for detecting signs of a sudden change in system state,
3 Apparatus for detecting signs of traffic congestion,
4 Apparatus for detecting pre-disease state,
10 Data generator,
11 Classifier,
12 Switch,
13 Dynamical network marker detector,
14 Switch,
20 Sample network calculator,
30 Sample specific network calculator,
40 Sample specific differential network calculator,
50 Local network extractor,
60 Gene expression probability calculator,
70 Network flow entropy calculator,
80 Differential network flow entropy calculator,
90 Temporal differential network flow entropy calculator,
100 Pre-disease state detector,
110 Switching means,
S10 Data generation step,
S11 Classification step,
S12 Switching step,
S11 Dynamical network marker detection step,
S12 Switching step,
S20 Sample network calculation step,
S30 Sample specific network calculation step,
S40 Sample specific differential network calculation step,
S50 Local network extraction step,
S60 Gene expression probability calculation step,
S70 Network flow entropy calculation step,
S80 Differential network flow entropy calculation step,
S90 Temporal differential network flow entropy calculation step,
S100 Pre-disease state detection step,
S110 Switching step.

**Claims**

1. An apparatus for detecting signs of a sudden change in system state, comprising:

   a classifier which classifies a plurality of nodes into a plurality of clusters based on the correlation of time-series changes of measurement data concerning a plurality of nodes constituting the target system; and
   a switch which selects a cluster that satisfies predefined selection conditions based on the time-series changes of measurement data of the nodes in each classified cluster and the correlation of time-series changes of measurement data among all the nodes and switches between detecting a node in the selected cluster as the dynamical network marker that can be used as predictors of sudden state changes and detecting a specific node that has been stored in advance as the dynamical network marker.

2. A method for detecting signs of a sudden change in system state, comprising:

a classifying step to classify a plurality of nodes into a plurality of clusters based on the correlation of time-series changes of measurement data concerning a plurality of nodes constituting the target system; and
a switching step to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

3. A program for detecting signs of a sudden change in system state which causes a computer to perform:

a classifying step to classify a plurality of nodes into a plurality of clusters based on the correlation of time-series changes of measurement data concerning a plurality of nodes constituting the target system; and
a switching step to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

4. A recording medium which records a program for detecting signs of a sudden change in system state which causes a computer to perform:

a classifying step to classify a plurality of nodes into a plurality of clusters based on the correlation of time-series changes of measurement data concerning a plurality of nodes constituting the target system; and
a switching step to select a cluster that satisfies predefined selection conditions based on the time-series changes of measurement data of the nodes in each classified cluster and the correlation of time-series changes of measurement data among all the nodes and to switch between detecting a node in the selected cluster as the dynamical network marker that can be used as predictors of sudden state changes and detecting a specific node that has been stored in advance as the dynamical network marker.

5. An apparatus for detecting signs of traffic congestion on the road, comprising:

a dynamical network marker detector which detects a dynamical network marker based on the dispersion of traffic volume at each node and the correlation of traffic volume among the nodes, where multiple points on the road are nodes; and
a switch which switches between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

6. A method for detecting signs of traffic congestion on the road, comprising:

a dynamical network marker detection step to detect a dynamical network marker based on the dispersion of traffic volume at each node and the correlation of traffic volume among the nodes, where multiple points on the road are nodes; and
a switching step to switch between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

7. A program for detecting signs of traffic congestion on the road which causes a computer to perform:

a dynamical network marker detection step to detect a dynamical network marker based on the dispersion of traffic volume at each node and the correlation of traffic volume among the nodes, where multiple points on the road are nodes; and

a switching step to switch between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

8. A recording medium which records a program for detecting signs of traffic congestion on the road which causes a computer to perform:

a dynamical network marker detection step to detect a dynamical network marker based on the dispersion of traffic volume at each node and the correlation of traffic volume among the nodes, where multiple points on the road are nodes; and
a switching step to switch between judging that there are signs of traffic congestion at the node detected as the dynamical network marker and detecting the dynamical network marker based on the dispersion of traffic volume and the correlation of traffic volume among the nodes only for the nodes that have been memorized in advance as likely to cause traffic congestion.

9. An apparatus for detecting pre-disease state which is the turning point from the normal state to the disease state, comprising:

a data generator which generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculator which calculates a reference sample network from the reference gene expression data and a perturbation sample network from the gene expression data for testing;
a sample specific network calculator which calculates a sample specific network which represents the relationship between the reference sample network and the perturbation sample network;
a sample specific differential network calculator which calculates a sample specific differential network from the sample specific network at the first time and the sample specific network at the second time after the first time;
a local network extractor which extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;
a gene expression probability calculator which calculates a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;
a differential network flow entropy calculator which calculates a differential network flow entropy from the local network flow entropy and the conditional network flow entropy;
a temporal differential network flow entropy calculator which calculates a temporal differential network flow entropy from the differential network flow entropy; and
a pre-disease state detector,
wherein the pre-disease state detector detects a pre-disease state when the difference between the temporal differential network flow entropy at the first time and the temporal differential network flow entropy at the second time is more than a predetermined value.

10. The apparatus for detecting pre-disease state according to claim 9,
wherein the gene expression probability is calculated assuming that the expression of each adjacent gene follows a normal distribution.

11. The apparatus for detecting pre-disease state according to claim 10,

wherein the reference gene expression data is an m-by-n matrix such that the (i, j) component represents the expression level of the i-th (i=1, ..., m) gene at a time point in the past or of a person in the j-th (j=1, ..., n) subject and wherein the gene expression data for testing is an m-by-n+1 matrix such that
(i, j) component represents the expression level of the i-th (i=1, ..., m) gene at a time point in the past or of a person in the j-th (j=1, ..., n) subject and (i, n+1) component represents the expression level of the i-th (i=1, ..., m) of the subject,
where the number of the time points or the number of the persons is n and the number of gene types is m.

**12.** The apparatus for detecting pre-disease state according to claim 11,

wherein the reference sample network is a Weighted Reference Network generated from the reference gene expression data and
wherein the perturbation sample network is a Weighted Perturbation Network generated from the gene expression data for testing.

**13.** The apparatus for detecting pre-disease state according to claim 12,
wherein the sample specific network is calculated by comparing the reference sample network with the perturbation sample network and leaving only the different edge.

**14.** The apparatus for detecting pre-disease state according to claim 13,

wherein the sample specific differential network is calculated by comparing
the sample specific network at the first time and the sample specific network at the second time and leaving only the different edge.

**15.** The apparatus for detecting pre-disease state according to claim 14,

wherein the gene expression probability p($x_{ikj}$) is: [equation 8]

$$p\left(x_{i_k j}\right) = \frac{\frac{1}{\sqrt{2\pi\sigma^2}} \int_{-\infty}^{x_{i_k j}} e^{\frac{-\left(t-\mu_{i_k}\right)^2}{2\left(\sigma_{i_k}\right)^2}} dt}{\sum_{j=1}^{n+1} \frac{1}{\sqrt{2\pi\sigma^2}} \int_{-\infty}^{x_{i_k j}} e^{\frac{-\left(t-\mu_{i_k}\right)^2}{2\left(\sigma_{i_k}\right)^2}} dt} \qquad (14)$$

where $x_{ikj}$ represents the (ik, j) component of the expression data of the i-th gene $g_i^k$ in the k-th local network and where $\mu_{ik}$ and $\sigma_{ik}$ are the mean deviation and the standard deviation of gene $g_i^k$, respectively.

**16.** The apparatus for detecting pre-disease state according to claim 15,

wherein the local network flow entropy $NFE_T(x_{ik})$ is: [equation 5]

$$NFE_T\left(x_{i_k}\right) = -\sum_{j=1}^{n+1} x_{i_k j} p\left(x_{i_k j}\right) \log\left(x_{i_k j} p\left(x_{i_k j}\right)\right) \qquad (11)$$

and wherein the conditional network flow entropy $NFE_T(x_{ik}/x_k)$ is:
[equation 9]

$$NFE_T\left(x_{i_k}/x_k\right) = -\sum_{j=1}^{n+1} x_{i_k j} p\left(x_{kj}, x_{i_k j}\right) \log\left(x_{i_k j} p\left(x_{i_k j}/x_{kj}\right)\right) \qquad (15)$$

where $x_{ik}$ is the expression data of the i-th gene $g_i^k$ in the k-th local network and
where $x_k$ is the expression data across genes in the k-th local network.

**17.** The apparatus for detecting pre-disease state according to claim 16,

wherein the differential network flow entropy $DNFE_T{}^k$ is:
[equation 12]

$$DNFE_T{}^k = \frac{\sum_{i=1}^{M_k}\left(NFE_T\left(x_{i_k}\right) - NFE_T\left(x_{i_k}/x_k\right)\right)}{M_k} \qquad (18)$$

where $M_k$ is the number of the adjacent genes.

18. The apparatus for detecting pre-disease state according to claim 17,

wherein the temporal differential network flow entropy $TNFE_T$ is:
[equation 13]

$$TNFE_T = \frac{\sum_{k=1}^{l} DNFE_T{}^k}{l} \qquad (19)$$

where $l$ is the number of the local networks.

19. The apparatus for detecting pre-disease state according to claim 18,
wherein the pre-disease state detector detects a pre-disease state when the temporal differential network flow entropy at the second time is twice or more than the temporal differential network flow entropy at the first time.

20. A method for detecting pre-disease state which is the turning point from the normal state to the disease state, comprising:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculation step to calculate a reference sample network from the reference gene expression data and a perturbation sample network from the gene expression data for testing;
a sample specific network calculation step to calculate a sample specific network which represents the relationship between the reference sample network and the perturbation sample network;
a sample specific differential network calculation step to calculate a sample specific differential network from the sample specific network at the first time and the sample specific network at the second time after the first time;
a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;
a gene expression probability calculation step to calculate a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;
a differential network flow entropy calculation step to calculate a differential network flow entropy from the local network flow entropy and the conditional network flow entropy;
a temporal differential network flow entropy calculation step to calculate a temporal differential network flow entropy from the differential network flow entropy; and
a pre-disease state detection step,
wherein the pre-disease state detection step detects a pre-disease state when the difference between the temporal differential network flow entropy at the first time and the temporal differential network flow entropy at the second time is more than a predetermined value.

21. A program for detecting pre-disease state which is the turning point from the normal state to the disease state which causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detection step detects a pre-disease state when the difference between the temporal differential network flow entropy at the first time and the temporal differential network flow entropy at the second time is more than a predetermined value.

22. A recording medium which records a program for detecting pre-disease state which is the turning point from the normal state to the disease state which causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detection step detects a pre-disease state when the difference between the temporal differential network flow entropy at the first time and the temporal differential network flow entropy at the second time is more than a predetermined value.

23. An apparatus for detecting pre-disease state which is the turning point from the normal state to the disease state, comprising:

a data generator which generates a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculator which calculates a reference sample network from the reference gene expression data and a perturbation sample network from the gene expression data for testing;
a sample specific network calculator which calculates a sample specific network which represents the relationship between the reference sample network and the perturbation sample network;
a sample specific differential network calculator which calculates a sample specific differential network from the sample specific network at the first time and the sample specific network at the second time after the first time;
a local network extractor which extracts a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;
a gene expression probability calculator which calculates a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;
a differential network flow entropy calculator which calculates a differential network flow entropy from the local network flow entropy and the conditional network flow entropy;
a temporal differential network flow entropy calculator which calculates a temporal differential network flow entropy from the differential network flow entropy; and
a pre-disease state detector,
wherein the pre-disease state detector comprises a switching means which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

24. A method for detecting pre-disease state which is the turning point from the normal state to the disease state, comprising:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;
a sample network calculation step to calculate a reference sample network from the reference gene expression data and a perturbation sample network from the gene expression data for testing;
a sample specific network calculation step to calculate a sample specific network which represents the relationship between the reference sample network and the perturbation sample network;
a sample specific differential network calculation step to calculate a sample specific differential network from the sample specific network at the first time and the sample specific network at the second time after the first time;
a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;
a gene expression probability calculation step to calculate a gene expression probability, which is the probability that each neighboring gene is expressed
a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detection step comprises a switching step which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

25. A program for detecting pre-disease state which is the turning point from the normal state to the disease state which causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detection step comprises a switching step which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

26. A recording medium which records a program for detecting pre-disease state which is the turning point from the normal state to the disease state which causes a computer to perform:

a data generation step to generate a reference gene expression data generated from biological samples collected at multiple time points in the subject's past or biological samples collected from a population including multiple persons and a gene expression data for testing which is the gene expression data of biological samples collected from subjects at a predetermined time added to this reference gene expression data;

a sample network calculation step to calculate a reference sample network from the reference gene expression data and a perturbation sample network from the gene expression data for testing;

a sample specific network calculation step to calculate a sample specific network which represents the relationship between the reference sample network and the perturbation sample network;

a sample specific differential network calculation step to calculate a sample specific differential network from the sample specific network at the first time and the sample specific network at the second time after the first time;

a local network extraction step to extract a local network that takes a structure in which a differential gene, which is a gene of which expression changes from the sample specific differential network to a predetermined degree or greater, is placed at the center and neighboring genes, of which correlation with the differential gene rises sharply above a given degree, are placed around the differential gene from the sample specific differential network;

a gene expression probability calculation step to calculate a gene expression probability, which is the probability that each neighboring gene is expressed

a network flow entropy calculator which calculates a local network flow entropy and a conditional network flow entropy under the condition that the differential gene expression has a given value, for each neighboring gene based on the gene expression probability;

a differential network flow entropy calculation step to calculate a differential network flow entropy from the local network flow entropy and the conditional network flow entropy;

a temporal differential network flow entropy calculation step to calculate a temporal differential network flow entropy from the differential network flow entropy; and

a pre-disease state detection step,

wherein the pre-disease state detection step comprises a switching step which switches between detecting a pre-disease state when the difference between the temporal differential network flow entropy at the first time and the temporal differential network flow entropy at the second time is more than a predetermined value and detecting a pre-disease state using local network that takes a structure in which a previously stored differential gene is placed at the center and neighboring genes, of which correlation with the differential gene rises sharply above a given degree, are placed around the differential gene from the sample specific differential network.

FIG. 1A

FIG. 1B

FIG. 1C

FIG. 1D

HEALTHY STATE

PRE-DISEASE STATE

DISEASE STATE

RECOVERABLE

DIFFICULT TO RECOVER

DISEASE WORSENING PROCESS

VALUE OF POTENTIAL FUNCTION

HEALTHY STATE

VALUE OF POTENTIAL FUNCTION

PRE-DISEASE STATE

VALUE OF POTENTIAL FUNCTION

DISEASE STATE

FIG. 2

FIG. 3

FIG. 4

# FIG. 5

FIG. 6

FIG. 7

# FIG. 8

# FIG. 9

FIG. 10

# FIG. 11

FIG. 12

# FIG. 13

FIG. 14

APPARATUS FOR DETECTING SIGNS OF
SUDDEN CHANGE IN SYSTEM STATE

NODE

CLASSIFIER

CLUSTER

SWITCH

DETECT NODE INCLUDED IN
SELECTED CLUSTER AS DNM

DETECT NODE STORED IN
ADVANCE AS DNM

EP 4 517 758 A1

FIG. 15

```
        ┌─────────────┐
        │    START    │
        └──────┬──────┘
               │
               ▼
   ┌───────────────────────┐
   │  CLASSIFICATION STEP   │ ～ S11
   └───────────┬───────────┘
               │
               ▼
   ┌───────────────────────┐
   │    SWITCHING STEP      │ ～ S12
   └───────────┬───────────┘
               │
               ▼
        ┌─────────────┐
        │     END     │
        └─────────────┘
```

# FIG. 16

APPARATUS FOR DETECTING SIGNS OF TRAFFIC CONGESTION

3

NODE

DNM検出手段  13

D N M

SWITCH  14

TRAFFIC CONGESTION AT NODE DETECTED AS DNM

DETECT DNM ONLY FOR NODES MEMORIZED AS LIKELY TO CAUSE TRAFFIC CONGESTION

EP 4 517 758 A1

FIG. 17

```
        ┌─────────────┐
        │    START    │
        └──────┬──────┘
               │
               ▼
   ┌───────────────────────────┐        S13
   │    DNM DETECTION STEP      │
   └───────────┬───────────────┘
               │
               ▼
   ┌───────────────────────────┐        S14
   │      SWITCHING STEP        │
   └───────────┬───────────────┘
               │
               ▼
        ┌─────────────┐
        │     END     │
        └─────────────┘
```

FIG. 18

**BIOLOGICAL SAMPLES**

→ **DATA GENERATOR** — 10

- REFERENCE GENE EXPRESSION DATA
- GENE EXPRESSION DATA FOR TESTING

**SAMPLE NETWORK CALCULATOR** — 20

- REFERENCE SAMPLE NETWORK
- PERTURBATION SAMPLE NETWORK

**SAMPLE SPECIFIC NETWORK CALCULATOR** — 30

- SAMPLE SPECIFIC NETWORK

**SAMPLE SPECIFIC DIFFERENTIAL NETWORK CALCULATOR** — 40

- SAMPLE SPECIFIC DIFFERENTIAL NETWORK

**LOCAL NETWORK EXTRACTOR** — 50

- LOCAL NETWORK

**APPARATUS FOR DETECTING PRE-DISEASE STATE** — 1

**PRE-DISEASE STATE DETECTOR** — 100

- TEMPORAL DIFFERENTIAL NETWORK FLOW ENTROPY

**TEMPORAL DIFFERENTIAL NETWORK FLOW ENTROPY CALCULATOR** — 90

- DIFFERENTIAL NETWORK FLOW ENTROPY

**DIFFERENTIAL NETWORK FLOW ENTROPY CALCULATOR** — 80

- LOCAL NETWORK FLOW ENTROPY
- CONDITIONAL NETWORK FLOW ENTROPY

**NETWORK FLOW ENTROPY CALCULATOR** — 70

- GENE EXPRESSION PROBABILITY

**GENE EXPRESSION PROBABILITY CALCULATOR** — 60

EP 4 517 758 A1

# FIG. 19A

# FIG. 19B

N HEALTHY PERSONS

N HEALTHY PERSONS    SUBJECT
($t = T$)

+

REFERENCE GENE
EXPRESSION DATA

REFERENCE GENE
EXPRESSION DATA

GENE EXPRESSION
DATA OF SUBJECT

GENE EXPRESSION
DATA FOR TESTING

SAMPLES

$s_1 \cdots s_j \cdots s_n$

$g_1$

GENE

$g_i$

$g_m$

Gene
expression
$x_{ij}$

SAMPLES

$s_1 \cdots s_j \cdots s_n \; S_{case_T}$

$g_1$

GENE

$g_i$

$g_m$

Gene
expression
$x_{ij}$

# FIG. 20A

REFERENCE GENE
EXPRESSION DATA

SAMPLES

$s_1 \cdots s_j \cdots s_n$

GENE
$g_1$
$\vdots$
$g_i$
$\vdots$
$g_m$

Gene expression $x_{ij}$

REFERENCE SAMPLE
NETWORK : WRN

# FIG. 20B

GENE EXPRESSION
DATA FOR TESTING

SAMPLES

$s_1 \cdots s_j \cdots s_n \; S_{case_T}$

GENE
$g_1$
$\vdots$
$g_i$
$\vdots$
$g_m$

Gene expression $x_{ij}$

PERTURBATION SAMPLE
NETWORK : WPN$_T$

# FIG. 21

FIG. 22

SAMPLE SPECIFIC NETWORK $(t = T)$ : $SST_T$

SAMPLE SPECIFIC NETWORK $(t = T - 1)$ : $SST_{T-1}$

SAMPLE SPECIFIC DIFFERENTIAL NETWORK : $SSDN_T$

EP 4 517 758 A1

# FIG. 23

SAMPLE SPECIFIC DIFFERENTIAL NETWORK : SSDN$_T$

NEIGHBORING GENE

DIFFERENTIAL GENE

LOCAL NETWORK

# FIG. 24

START → BIOLOGICAL SAMPLES → **DATA GENERATOR** (S10)

REFERENCE GENE EXPRESSION DATA / GENE EXPRESSION DATA FOR TESTING → **SAMPLE NETWORK CALCULATOR** (S20)

REFERENCE SAMPLE NETWORK / PERTURBATION SAMPLE NETWORK → **SAMPLE SPECIFIC NETWORK CALCULATOR** (S30)

SAMPLE SPECIFIC NETWORK → **SAMPLE SPECIFIC DIFFERENTIAL NETWORK CALCULATOR** (S40)

SAMPLE SPECIFIC DIFFERENTIAL NETWORK → **LOCAL NETWORK EXTRACTOR** (S50)

LOCAL NETWORK → **GENE EXPRESSION PROBABILITY CALCULATOR** (S60)

GENE EXPRESSION PROBABILITY → **NETWORK FLOW ENTROPY CALCULATOR** (S70)

LOCAL NETWORK FLOW ENTROPY / CONDITIONAL NETWORK FLOW ENTROPY → **DIFFERENTIAL NETWORK FLOW ENTROPY CALCULATOR** (S80)

DIFFERENTIAL NETWORK FLOW ENTROPY → **TEMPORAL DIFFERENTIAL NETWORK FLOW ENTROPY CALCULATOR** (S90)

TEMPORAL DIFFERENTIAL NETWORK FLOW ENTROPY → **PRE-DISEASE STATE DETECTION STEP** (S100) → END

EP 4 517 758 A1

# FIG. 25

APPARATUS FOR DETECTING PRE-DISEASE STATE — 4

BIOLOGICAL SAMPLES

DATA GENERATOR ~ 10

REFERENCE GENE EXPRESSION DATA | GENE EXPRESSION DATA FOR TESTING

SAMPLE NETWORK CALCULATOR ~ 20

REFERENCE SAMPLE NETWORK | PERTURBATION SAMPLE NETWORK

SAMPLE SPECIFIC NETWORK CALCULATOR ~ 30

SAMPLE SPECIFIC NETWORK

SAMPLE SPECIFIC DIFFERENTIAL NETWORK CALCULATOR ~ 40

SAMPLE SPECIFIC DIFFERENTIAL NETWORK

LOCAL NETWORK EXTRACTOR ~ 50

LOCAL NETWORK

GENE EXPRESSION PROBABILITY CALCULATOR ~ 60

GENE EXPRESSION PROBABILITY

NETWORK FLOW ENTROPY CALCULATOR ~ 70

LOCAL NETWORK FLOW ENTROPY | CONDITIONAL NETWORK FLOW ENTROPY

DIFFERENTIAL NETWORK FLOW ENTROPY CALCULATOR ~ 80

DIFFERENTIAL NETWORK FLOW ENTROPY

TEMPORAL DIFFERENTIAL NETWORK FLOW ENTROPY CALCULATOR ~ 90

TEMPORAL DIFFERENTIAL NETWORK FLOW ENTROPY

PRE-DISEASE STATE DETECTOR 110

SWITCHING MEANS

100

# FIG. 26

EP 4 517 758 A1

<div align="center">

## INTERNATIONAL SEARCH REPORT

</div>

| International application No. |
| --- |
| **PCT/JP2023/014370** |

**A.    CLASSIFICATION OF SUBJECT MATTER**

*G16B 20/00*(2019.01)i; *G16B 25/10*(2019.01)i; *G16H 50/30*(2018.01)i
FI:    G16B20/00; G16H50/30; G16B25/10

According to International Patent Classification (IPC) or to both national classification and IPC

**B.    FIELDS SEARCHED**

Minimum documentation searched (classification system followed by classification symbols)

G16B20/00; G16B25/10; G16H50/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2018/207925 A1 (JAPAN SCIENCE & TECH AGENCY) 15 November 2018 (2018-11-15) claim 1, paragraphs [0013]-[0030], [0046]-[0052] | 1-26 |
| A | JP 2021-113695 A (MITSUBISHI HEAVY IND LTD) 05 August 2021 (2021-08-05) paragraphs [0049]-[0063] | 1-26 |
| A | JP 2014-83194 A (JAPAN SCIENCE & TECHNOLOGY AGENCY) 12 May 2014 (2014-05-12) paragraphs [0043], [0046], [0056], [0094]-[0100] | 1-26 |
| E, A | JP 2023-57492 A (MITSUBISHI HEAVY INDUSTRIES, LTD.) 21 April 2023 (2023-04-21) claims 1-3, paragraphs [0029]-[0059] | 1-26 |

☐ Further documents are listed in the continuation of Box C.    ☑ See patent family annex.

| | |
| --- | --- |
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| **07 June 2023** | **20 June 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
**Information on patent family members**

International application No.

**PCT/JP2023/014370**

| Patent document cited in search report | | | Publication date (day/month/year) | Patent family member(s) | Publication date (day/month/year) |
|---|---|---|---|---|---|
| WO | 2018/207925 | A1 | 15 November 2018 | US      2021/0158899      A1 claim 1, paragraphs [0026]-[0059], [0078]-[0091] CN      110603592      A | |
| JP | 2021-113695 | A | 05 August 2021 | US      2021/0223141      A1 paragraphs [0074]-[0088] CN      113139244      A | |
| JP | 2014-83194 | A | 12 May 2014 | US      2015/0302165      A1 paragraphs [0073], [0076], [0091], [0125]-[0131] KR  10-2015-0099726      A CN      105009130      A EP      2913770      A1 | |
| JP | 2023-57492 | A | 21 April 2023 | (Family: none) | |

Form PCT/ISA/210 (patent family annex) (January 2015)

## REFERENCES CITED IN THE DESCRIPTION

### Patent documents cited in the description

- JP 2014064515 A
- JP 2014083194 A
- WO 2018207925 A

### Non-patent literature cited in the description

- Self-organized patchiness in asthma as a prelude to catastrophic shifts. **JOSE G. VENEGAS** ; **TILO WINKLER** ; **GUIDO MUSCH** ; **MARCOS F. VIDAL MELO** ; **DOMINICK LAYFIELD** ; **NORA TGAVALE-KOS** ; **ALAN J. FISCHMAN** ; **RONALD J. CALL-AHAN** ; **GIACOMO BELLANI** ; **R. SCOTT HARRIS**. Nature. Nature Publishing Group, 2005, vol. 434, 777-782
- Prediction of epileptic seizures: are nonlinear methods relevant?. **PATRICK E. MCSHARRY** ; **LEONARD A. SMITH** ; **LIONEL TARASSENKO**. Nature Medicine. Nature Publishing Group, 2003, vol. 9, 241-242
- Transition models for change-point estimation in logistic regression. **ROBERTO PASTOR-BARRIU-SO** ; **ELISEO GUALLAR** ; **JOSEF CORESH**. Statistics in Medicine. Wiley-Blackwell, 2003, vol. 22, 1141-1162
- Hearing preservation after gamma knife stereotactic radiosurgery of vestibular schwannoma. **PAEK SH et al.** Cancer. Wiley-Blackwell, 2005, vol. 104, 580-590
- Pituitary Apoplexy. **LIU, J.K.** ; **ROVIT, R.L.** ; **COULDWELL, W.T.** Seminars in Neurosurgery. Thieme, 2001, vol. 12, 315-320
- **G.TANAKA** ; **K.TSUMOTO** ; **S.TSUJI** ; **K.AIHARA**. Bifurcation analysis on a hybrid systems model of intermittent hormonal therapy for prostate cancer. *Physica D: Nonlinear Phenomena*, 15 October 2008, vol. 237 (20), 2616-2627
- **MD ABDUS SAMAD KAMAL1** ; **MAKITO OKU** ; **TOMOHISA HAYAKAWA** ; **JUN-ICHI IMURA** ; **KAZUYUKI AIHARA**. Early Detection of a Traffic Flow Breakdown in the Freeway Basedon Dynamical Network Markers. *International Journal of Intelligent Transportation Systems Research*, 2020, vol. 18 (422), 435